(19) Europäisches Patentamt
European Patent Office
Office européen des brevets

(11) Numéro de publication: **0 253 711**
**B1**

(12) **FASCICULE DE BREVET EUROPEEN**

(45) Date de publication du fascicule du brevet: **23.05.90**

(21) Numéro de dépôt: **87401551.4**

(22) Date de dépôt: **02.07.87**

(51) Int. Cl.⁵: **C 07 D 513/04,**
C 07 D 519/00, A 61 K 31/435
// (C07D513/04, 277:00, 209:00),(C07D519/00, 513:00, 495:00)

(54) **Nouveaux dérivés du 1H,3H-Pyrrolo 1,2-cr thiazole, leur préparation et les Compositions pharmaceutiques qui les contiennent.**

(30) Priorité: **04.07.86 FR 8609728**

(43) Date de publication de la demande:
**20.01.88 Bulletin 88/03**

(45) Mention de la délivrance du brevet:
**23.05.90 Bulletin 90/21**

(84) Etats contractants désignés:
**AT BE CH DE ES FR GB GR IT LI LU NL SE**

(56) Documents cités:
**EP-A-0 115 979**

(73) Titulaire: **RHONE-POULENC SANTE**
**20, avenue Raymond Aron**
**F-92160 Antony (FR)**

(72) Inventeur: **Fabre, Jean-Louis**
**9, rue fagon**
**F-75013 Paris (FR)**
Inventeur: **James, Claude**
**31 bis, avenue Gambetta**
**F-75020 Paris (FR)**
Inventeur: **Lavé, Daniel**
**72 boulevard de la Villette**
**F-75019 Paris (FR)**

(74) Mandataire: **Pilard, Jacques et al**
**RHONE-POULENC SANTE, Service Brevets, 20 Avenue Raymond Aron**
**F-92165 Antony Cédex (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**EP 0 253 711 B1**

Courier Press, Leamington Spa, England.

**Description**

La présente invention concerne de nouveaux dérivés du 1H,3H-pyrrolo [1,2-c] thiazole de formule générale:

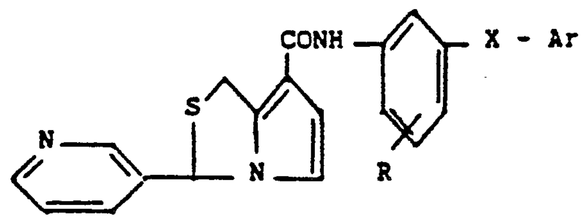

(I)

dans laquelle R représente un atome d'hydrogène ou d'halogène ou un radical alcoyloxy, X représente un atome d'oxygène ou de soufre ou un radical imino, carbonyle, carbonylméthylène, ou vinylènecarbonyle, ou bien représente un radical méthylène et Ar représente un radical phényle, naphtyle, pyridyle, thiényle, ces radicaux pouvant être non substitués ou bien porter un substituant choisi parmi les atomes d'halogène ou les radicaux alcoyle ou alcoyloxy, étant entendu que les radicaux alcoyle et portions alcoyle contiennent 1 à 4 atomes de carbone en chaïne droite ou ramifiée et que l'invention concerne les produits racémiques, les énantiomères dus à la présence d'un carbone asymétrique en position 3 du cycle pyrrolothiazole et les mélanges de ces énantiomères, ainsi que leurs sels pharmaceutiquement acceptables.

Selon l'invention, les produits de formule générale (I) peuvent être préparés par action d'une amine de formule générale:

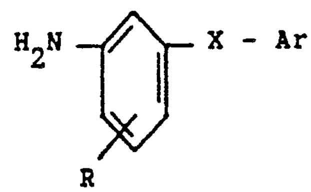

(II)

dans laquelle R, X et Ar sont définis comme précédement sur un acide un acide de formule:

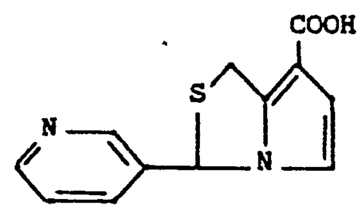

(III)

ou un dérivé réactif de cet acide.

Il est, en effet, particulièrement avantageux d'utiliser l'acide de formule (III) sous und forme activée telle que le chlorure d'acide ou de le faire réagir avec le N,N'-carbonyl-diimidazole ou un chloroformiate d'alcoyle avant de condenser l'amine de formule générale (II).

Généralement il est préférable d'employer le chlorure d'acide et d'effectuer la réaction dans un solvant organique tel que le chloroforme, le chlorure de méthylène ou le dioxanne à une température comprise entre 0°C et la température de reflux du mélange réactionnel, en présence d'un accepteur d'acide comme la triéthylamine.

L'acid de formule (III) racémique peut être préparé selon la méthode décrite dans le brevet européen n° 0 115 979.

Les amines de formule générale (II) peuvent être préparées par application ou adaptation des méthodes déjà décrites dans la littérature.

La présence en positon 3 du cycle pyrrolo [1,2-c] thiazole d'une carbone asymétrique fait que les produits de formule générale (I) selon l'invention peuvent exister sous forme de racémiques ou sous forme d'énantiomères. Généralement le procédé decrit précédemment conduit aux produits racémiques mais il est entendu que l'on peut obtenir directement les énantiomères correspondants si l'on met en oeuvre le procédé en opérant avec un acide de formule (III) optiquement actif.

L'acide de formule (III) optiquement actif peut être préparé selon l'un ou l'autre des procédés suivants:

A — Premier procédé: On saponifie un ester optiquement actif de pouvoir rotatoire correspondant de formule générale:

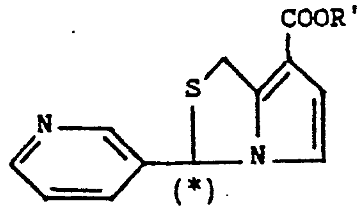

(IV)

dans laquelle R′ représente un radical alcoyle contenant 1 à 4 atomes de carbone en chaîne droite ou ramifiée et le symbole * représente le signe + ou le signe − selon que le produit mis en oeuvre est dextrogyre ou lévogyre.

Généalement la saponification s'effectue par toute méthode douce connue de l'homme du métier pour transformer un ester en acide sans racémiser les centres chiraux présents dans la molécule. Il est particulièrement avantageux d'effectuer la saponification au moyen d'un hydroxyde de métal alcalin tel que la soude ou la potasse à une température comprise entre 20 et 50°C.

L'ester de formule générale (IV) peut être obtenu en faisant réagir le produit de réaction du chlorure de p-toluène sulfonyle, de la triéthylamine et de l'acide de formule:

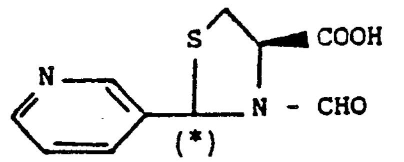

(V)

dans laquelle le symbole * a la même signification que dans l'ester (IV) correspondant défini précédemment sur le produit de réaction de la triéthylamine et d'un dichloro-2,3 propionate d'alcoyle de formule générale:

$$ClCH_2-\underset{\displaystyle Cl}{\underset{|}{C}H}-COOR' \qquad (VI)$$

dans laquelle R′ est défini comme précédemment, au sein d'un solvant organique tel que le dichloro-1,2 éthane ou le chlorure de méthylène à un température comprise entre 20°C et la température de reflux du mélange réactionnel.

L'acide de formule générale (V) peut être obtenu par action d'un mélange d'acide formique et d'anhydride acétique sur un acide de formule:

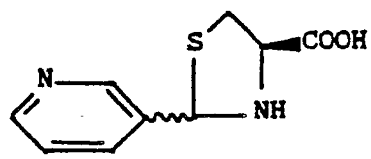

(VII)

et séparation ultérieure des formes dextrogyre et lévogyre en opérant selon les méthodes habituelles, par exemple par recristallisation et//ou formation de sels avec des bases optiquement actives comme l'α-méthylbenzylamine, séparation de ses sels et libération de l'acide correspondant.

B — Deuxième procédé: On sépare les énantiomères de l'acide de formule (III) par toute méthode connue de l'homme du métier, notamment par formation d'un sel avec une base optiquement active telle que les formes optiquement actives de l'α-méthylbenzylamine, recristallisations du sel obtenu et décomposition de celui-ci par un acide tel que l'acide chlorhydrique.

Selon l'invention, les produits de formule générale (I), dextrogyres, lévogyres ou racémiques peuvent également être préparés en faisant réagir le produit de réaction du chlorure de p-toluènesulfonyle, de la triéthylamine et de l'acide dextrogyre, lévogyre ou racémique correspondant de formule (V) sur le produit de réaction de la triéthylamine et d'un produit de formule générale:

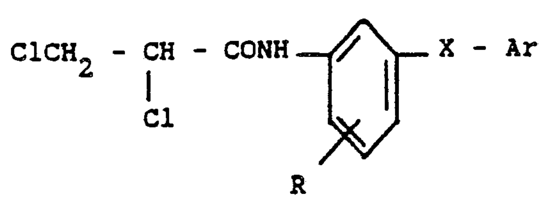

(VIII)

dans laquelle les symboles sont définis comme précédemment.

On opère généralement au sein d'un solvant organique tel que le dichloro-1,2 éthane ou le chlorure de méthylène à un température comprise entre 20°C et la température de reflux du mélange réactionnel.

Les produits de formule générale (VIII) peuvent être préparés par application ou adaptation de méthodes connues dans la littérature, nottamment par action du chlorure de dichloro-2,3 propionyle sur une amine de formule générale (II) définie comme précédemment, en opérant dans le toluène à une température comprise entre 20°C et la température de reflux du mélange réactionnel.

Il est entendu pour l'homme du métier que, pour la mise en oeuvre des procédés décrits précédemment, il peut être nécessaire d'introduire des groupements protecteurs au niveau de certaines fonctions présentes dans les radicaux R et Ar des différents produits mis en oeuvre. Le groupement protecteur pourra être ensuite éliminé au moment le plus propice de la synthèse. Ainsi, lorsque dans le radical Ar et/ou R est présente une fonction amino ou alcoylamino, celle-ci pourra être protégée par

exemple par un radical tert-butyloxycarbonyle puis libérée après réaction au moyen d'un acide aqueux, par exemple au moyen d'une solution aqueuse d'acide chlorhydrique, ou mieux au moyen d'une solution acétique de gaz chlorhydrique. Lorsque, dans le radical Ar et/ou R est présente une fonction hydroxy, celle-ci pourra être avantageusement protégée sous forme de radical tétrahydropyranyloxy ou méthoxyméthyloxy, puis libérée après réaction par hydrolyse. Lorsque, dans le radical Ar et/ou R est présente une fonction carboxylique, celle-ci pourra être avantageusement protégée sous forme d'ester d'alcoyle qui pourra être saponifié pour donner l'acide correspondant selon les méthodes habituelles.

Les nouveaux produits de formule générale (I) peuvent être transformés en sel d'addition avec les acides par action d'un acide dans un solvant organique tel qu'un alcool, une cétone, un éther ou un solvant chloré. Le sel précipite, généralement après concentration de sa solution; il est séparé par filtration ou décantation.

Les nouveaux produits de formule générale (I) contenant dans leur molécule un groupement acide peuvent être peuvent être transformés en sels métalliques ou en sels d'addition avec les bases azotées, par toute méthode connue de l'homme du métier pour effectuer cette salification sans toucher au reste de la molecule.

Lorsque, dans la présente description, on se réfère à un produit déterminé indentifié par son nom chimique, il est entendu qu'en l'absence de spécifications particulières concernant la nature des isomères, on se réfère toujours au produit racémique correspondant.

Les nouveaux produits selon l'invention ainsi que leurs sels d'addition présentent des propriétés pharmacologiques intéresantes associées à une faible toxicité. Ils se sont montrés actifs à des concentrations inhibitrices ($CI_{50}$) comprises entre 1 et 1000 nM dans le test d'antagonisme de la fixation du [$^3$H] O-octadécyl-1-O-acétyl-2 ns-glycérophosphorylcholine-3 (P.A.F.-acéther tritié) sur ses sites récepteurs des plaquettes sanguines selon la technique suivante:

a) Préparation des plaquettes lavées de lapin. Des lapins mâles néozélandais (hybrid HY 2000) d'un poids de 2,5 kg environ sont ponctionnés à l'artère de l'oreille. Le sang est recueilli sur un mélange d'acide citrique (1,9 mM), de citrate trisodique (9 mM), de phosphate monosodique (1,75 mM) et de dextrose (5,6 mM). Le sang est centrifugé à 120 g pendant 20 minutes à 15°C. On obtient ainsi le plasma riche en plaquettes (PRP). Ce plasma est centrifugé à 1000 g pendant 15 minutes à 15°C. Le culot plaquettaire ainsi obtenu est lavé une première fois par une solution de Tyrode modifiée contenant 0,35% de sérumalbumine bovine (BSA), 2 mMole par litre de $MgCl_2$, 0,2 mMole par litre d'EGTA, puis par une solution de Tyrode sans EGTA. Les plaquettes sont finalement remises en suspension dans un tampon d'essai (tampon A) ayant la composition suivante: NaCl (140 mM), KCl (2,7 mM), $NaH_2PO_4$ (0,4 mM), $MgCl_2$ (2 mM), $NaHCO_3$ (12 mM), tampon Tris, HCl (10 mM), dextrose (6,2 mM) et BSA (0,25%). La concentration finale de la suspension est ajustée à $4.10^8$ plaquettes/cm$^3$ au moyen de ce tampon.

b) Mise en oeuvre du test proprement dit.

Dans un tube de 5 cm$^3$, on introduit successivement le tampon A décrit précédemment, le produit à étudier, le PAF-acéther tritié (0,5 nMole — Activité spécifique : 80 Ci/mMole) et les plaquettes obtenues comme decrit précédemment ($0,5.10^8$ plaquettes), de manière à obtenir un volume final de 0,5 cm$^3$ et on laisse le mélange incuber pendant une heure à 20°C. On ajoute alors 2 cm$^3$ de tampon A refroidi à 4°C, filtre rapidement le contenu du tube sur filtre WHATMAN GF/C (marque déposée) et rinse le tube très rapidement 3 fois avec 2 cm$^3$ de tampon A refroidi à 4°C. Le filte est séché et placé dans une fiole contenant 4,5 cm$^3$ de liquide scintillant READY SOLV. MP (N.D. BECKMAN) et la radiactivité est mesurée au moyen d'un compteur universel RACK BETA 1218 LKB. On détermine ainsi la radioactivité liée totale. La fixation spécifique du PAF-acéther tritié est déterminée en retranchant de la radioactivité liée totale, la radioactivité restée sur le filtre après addition de 10µMole de N-(méthoxy-3 phényl) (pyridyl-3)-3 1H,3H-pyrrolo [1,2-c] thiazolecarboxamide-7. Pour chaque produit à étudier, on reproduit l'essai 3 fois à des concentrations croissantes allant de $10^{-10}$ à $10^{-4}$M. On détermine graphiquement la $CI_{50}$ pour chaque produit par analyse en log Probit de la courbe d'inhibition.

On sait que le PAF-acéther intervient dans un grand nombre de maladies et de troubles tels que les réactions allergiques (asthme ou bronchite) ou les réactions inflammatoires de muqueuses gastriques et intestinales d'origines diverses et notamment les réactions inflammatoires dues aux irradiations et aux chocs endotoxiniques ainsi que les troubles liés à l'agrégation des plaquettes. Le PAF-acéther libéré au cours des ces désordres se fixe sur des récepteurs spécifiques de ce médiateur. Le test de liaison aux récepteurs des plaquettes sanguines décrit précédemment est l'un des modèles expérimentaux possibles pour étudier l'aptitude des produits à se fixer à ces récepteurs.

Les produits selon l'invention déplacent le PAF-acéther de ses sites de liaison. Ils entrent donc en compétition avec lui et en antagonisent les effets. Ainsi il est prévisible que les produits selon l'invention auront un rôle thérapeutique dans le traitement des maladies et des états énumérés ci-dessus.

On connait déjà par le brevet européen 0 115 979 des pyrrolothiazoles qui ont une certaine action inhibitrice vis-à-vis du PAF-acéther mais les produits selon, la présente invention se fixent aux récepteurs des plaquettes à des doses beaucoup plus faibles et sont donc plus aptes à inhiber les effets du PAF-acéther.

Par ailleurs, les produits selon l'invention présentent une toxicité faible. Leur $DL_{50}$ est généralement comprise entre 300 et 900 mg/kg chez la souris/par voie orale.

Sont plus particulièrement intéressants les produits de formule générale (I) dans laquelle R représente

un atome d'hydrogène, X représente un atome d'oxygène ou un radical carbonyle et Ar représente un radical phényle ou pyridyle, ces radicaux pouvant être non substitués ou substitués par un atome d'halogène ou un radical alcoyle ou alcoyloxy, étant entendu que les radicaux alcoyle et portions alcoyle contiennent 1 à 4 atomes de carbone en chaîne droite ou ramifiée et que les produits concernés sont les produits racémiques, les énantiomères dus à la présence d'un carbone asymétrique en position 3 du cycle pyrrolothiazole et les mélanges de ces énantiomères.

Sont d'un intérêt tout particulier les produits suivants:

(+)N-(benzoyl-3 phényl) (pyridyl-3)-3 1H,3H-pyrrolo [1,2-c] thiazolecarboxamide-7
N-(benzoyl-3 phényl) (pyridyl-3)-3 1H,3H-pyrrolo [1,2-c] thiazolecarboxamide-7
N-(phénoxy-3 phényl) (pyridyl-3)-3 1H,3H-pyrrolo [1,2-c] thiazolecarboxamide-7
N-[(méthyl-2 phénoxy)-3 phényl] (pyridyl-3)-3 1H,3H-pyrrolo [1,2-c] thiazolecarboxamide-7
N-[(pyridyl-3 oxy)-3 phényl] (pyridyl-3)-3 1H,3H-pyrrolo [1,2-c] thiazolecarboxamide-7
N-[(méthyl-3 phénoxy)-3 phényl] (pyridyl-3)-3 1H,3H-pyrrolo [1,2-c] thiazolecarboxamide-7
N-[(méthyl-3 benzoyl)-3 phényl] (pyridyl-3)-3 1H,3H-pyrrolo [1,2-c] thiazolecarboxamide-7
N-[(chloro-4 phénoxy)-3 phényl] (pyridyl-3)-3 1H,3H-pyrrolo [1,2-c] thiazolecarboxamide-7
N-(nicotinoyl-3 phényl) (pyridyl-3)-3 1H,3H-pyrrolo [1,2-c] thiazolecarboxamide-7

D'une façon générale, les produits selon l'invention qui sont les plus intéressants sont ceux qui se présentent sous la forme racémique et les isomères optiques de forme dextrogyre.

Pour l'emploi thérapeutique, il peut être fait usage des nouveaux produits de formule générale (I), tels quels ou, les cas échéant, à l'état de sels pharmaceutiquement acceptables, c'est-à-dire non toxiques aux doses d'utilisation.

Come sels pharmaceutiquement acceptables peuvent être cités les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophillineacétates, salicylates, phénolphtalinates, méthylène-bis-β-oxynaphtoates ou des dérivés de substitution de ces composés. Lorqu'ils peuvent exister, on peut encore citer les sels avec les métaux alcalins tels que les sels de sodium, potassium ou lithium, avec les métaux alcalino-terreux tels que les sels de calcium ou de magnésium, et les sels d'addition avec les bases organiques tels que les sels d'éthanolamine ou de lysine.

Les exemples suivants, donnés à titre non limitatif, montrent comment l'invention peut être mise en pratique.

Exemple 1

A une solution de 2,8 g de phénoxy-3 aniline et de 3,05 g de triéthylamine dans 100 $cm^3$ de dioxanne, chauffée à une température voisine de 60°C, on ajoute en 5 minutes, à une température comprise entre 60°C et 68°C, 4,5 g de chlorhydrate de chloroformyl-7-(pyridyl-3)-3 1H,3H-pyrrolo [1,2-c] thiazole. La suspension obtenue est chauffée sous agitation à une température voisine de 100°C pendant 6 heures et 15 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 250 $cm^3$ de chlorure de méthylène et la solution obtenue est lavée 2 fois par 200 $cm^3$ au total d'eau distillée, 2 fois par 200 $cm^3$ au total d'une solution aqueuse de soude 4N et 2 fois par 200 $cm^3$ au total d'eau distillée puis séchée sur du sulfate de magnésium anhydre, additionée de 0,5 g de noir décolorant, filtrée et concentrée à sec sous pression réduite (20 mm de mercure; 2,7 kPa) à une température voisine de 50°C. On obtient ainsi 6,7 g de produit brut. Ce produit est dissous dans 25 $cm^3$d'isopropanol bouillant. La solution obtenue est additionnée de 0,5g de noir décolorant et filtrée à chaud. Le filtrat est refoidi à une température voisine de 4°C pendant 3 heures. Les cristaux apparus sont séparés par filtration, lavés 4 fois par 20 $cm^3$ au total d'isopropanol puis 3 fois par 75 $cm^3$ au total d'éther diéthylique et séchés sous pression réduite (20 mm de mercure; 2,7 kPa) à une température voisine de 20°C en présence de potasse en pastilles. On obtient ainsi 2,1 g de N-(phénoxy-3 phényl) (pyridyl-3)-3 1H,3H-pyrrolo [1,2-c] thiazolecarboxamide-7 sous forme de cristaux crème fondant à 144°C.

La phénoxy-3 aniline peut être préparée selon la méthode décrite par F. ULLMANN et P. SPONAGEL, Annalen, *350,* 83 (1906).

Le chlorhydrate de chloroformyl-7 (pyridyl-3)-3 1H,3H-pyrolo [1,2-c] thiazole est préparé selon la méthode décrite dans le brevet européen n° 0 115 979.

Exemple 2

A une solution de 16,1 g d'amino-3 benzophénone et de 16,5 g de triéthylamine dans 420 $cm^3$ de dioxanne chauffée à une température voisine de 60°C, on ajoute en 5 minutes à une température comprise entre 60 et 72°C, 24,5 g de chlorhydrate du chlorure d'acide provenant de l'acide (+) (pyridyl-3)-3 1H,3H-pyrrolo [1,2-c] thiazole carboxylique-7. La suspension obtenue est chauffée sous agitation à une température voisine de 100°C pendant 6 heures et 30 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 750 $cm^3$ d'acétate d'éthyle. La solution obtenue est lavée 3 fois par 800 $cm^3$ au total d'eau distillée, 2 fois par 600 $cm^3$ au total d'une solution aqueuse saturée de bicarbonate de sodium et 2 fois par 600 $cm^3$ au total d'eau distillée puis séchée sur du sulfate de magnésium anhydre, additionnée de 0,5 g de noir décolorant, filtrée et concentrée à sec sous pression

réduite (20 mm de mercure; 2,7 kPa) à une température voisine de 60°C. On obtient ainsi 36 g de produit brut que l'on dissout dans 200 $cm^3$ d'un mélange bouillant d'éthanol et d'eau (85—15 en volumes). La solution obtenue est additionnée de 0,5 g de noir décolorant et filtrée à chaud. Le filtrat est refroidi à une température voisine de 20°C pendant 3 heures. Les cristaux aparaus sont séparés par filtration, lavés 3 fois par 75 $cm^3$ au total d'un melange d'éthanol et d'eau (85—15 en volumes) et 4 fois par 200 $cm^3$ au total d'ether diéthylique puis séchés sous pression réduite (20 mm de mercure; 2,7 kPa) à une température voisine de 20°C en présence de potasse en pastilles. On obtient ainsi 20,7 g de (+)N-(benzoyl-3 phényl) (pyridyl-3)-3 1H,3H-pyrrolo [1,2-c] thiazolecarboxamide-7 à l'état d'hydrate sous forme de cristaux crème fondant à 109°C.

$$[\alpha]_D^{20} + 87,5° \pm 1° \text{ (c = 1,02; diméthylformamide).}$$

Le chlorhydrate du chlorure d'acide provenant de l'acide (+) (pyridyl-3)-3 1H,3H pyrrolo [1,2-c] thiazole carboxylique-7 est préparé de la façon suivante : Une suspension de 20,9 g d'acide (+) (pyridyl-3)-3 1H,3H-pyrrolo [1,2-c] thiazolecarboxylique-7 dans un mélange de 52,1 g de chlorure de thionyle, de 0,1 $cm^3$ de diméthylformamide et de 290 $cm^3$ de dichloro-1,2 éthane est chauffée à une tempèrature voisine de 80°C pendant 3 heures. Après refroidissement à une température voisine de 20°C, les cristaux sont séparés par filtration, lavés 3 fois par 150 $cm^3$ au total de dichloro-1,2 éthane, 3 fois par 150 $cm^3$ d'éther diéthlique puis séchés sous pression réduite (20 mm de mercure; 2,7 kPa) à une température voisine de 20°C On obtient ainsi 24,5 g de chlorhydrate du chlorure d'acide provenant de l'acide (+) (pyridyl-3)-3 1H,3H-pyrrolo [1,2-c] thiazolecarboxylique-7 sous forme de cristaux crème a 175°C.

L'acide (+) (pyridyl-3)-3 1H,3H-pyrrolo [1,2-c] thiazolecarboxylique-7 peut être obtenu de la façon suivante selon l'un ou l'autre des procédés suivants:

A — Premier procédé: On chauffe à une température voisine de 40°C pendant 14 heures une solution de 19,5 g de (+) (pyridyl-3)-3 1H,3H-pyrrolo [1,2-c] thiazolecarboxylate-7 d'éthyle et de 11,9 g de potasse en pastilles dans um mélange de 70 $cm^3$ d'éthanol et de 70 $cm^3$ d'eau distillée. Le solvant est évaporé sous pression réduite (20 mm de mercure; 2,7 kPa) à une température voisine de 40°C. Le résidu est dissous dans 200 $cm^3$ d'eau distillée et la solution obtenue est amenée à un pH voisin de 4 par addition de 250 $cm^3$ d'une solution aqueuse d'acide chlorhydrique N et est agitée à une température voisine de 20°C pendant 1 heure. Les cristaux apparus sont séparés par filtration, lavés 5 fois par 250 $cm^3$ au total d'eau distillée, 5 fois par 150 $cm^3$ au total d'éthanol et 3 fois par 90 $cm^3$ au total d'éther diéthylique puis séchés sous pression réduite (20 mm de mercure; 2.7 kPa) à une température voisine de 20°C en présence de potasse en pastilles. On obtient 14,1 g de produit brut fondant à 210°C. Ce produit est dissous dans 420 $cm^3$ d'éthanol bouillant; la solution obtenue est additonnée de 0,5 g de noir décolorant et filtrée à chaud. Le filtrat est refroidi à une température voisine de 4°C pendant 16 heures. Les cristaux apparus sont séparés par filtration, lavés 3 fois par 90 $cm^3$ au total d'éthanol et 3 fois par 150 $cm^3$ au total d'éther diéthylique puis séchés sous pression réduite (20 mm de mercure; 2,7 kPa) à une température voisine de 20°C. On obtient ainsi (10, 3 g d'acide (+) (pyridyl-3)-3 1H,3H-pyrrolo [1,2-c] thiazolecarboxylique-7 sous forme de cristaux crème fondant à 210°C.

$$[\alpha]_D^{20} + 163° \pm 1,6° \text{ (c = 1,08; soude N).}$$

Le (+) (pyridyl-3)-3 1H,3H-pyrrolo [1,2-c] thiazolecarboxylate-7 d'éthyle peut être préparé de la façon suivanté: A une suspension de 23,8 g d'acide N-formyl (pyridyl-3)-2 thiazolidinecarboxylique-4-(2R,4R) dans 90 $cm^3$ de dichloro-1,2 éthane, on ajoute en 2 minutes, à une température comprise entre 20 et 27°C, 11,2 g de triéthylamine. La suspension obtenue est agitée à une température voisine de 20°C pendant 1 heure et la solution obtenue est ajoutée en 50 minutes à une température voisine de 20°C, à une solution de 21 g de chlorure de paratoluènesulfonyle dans 110 $cm^3$ de dichloro-1,2 éthane. On obtient une solution trouble (solution A). Par ailleurs, à une solution de 18,6 g de dichloro-2,3 propionate d'éthyle dans 100 $cm^3$ de dichloro-1,2 éthane, on ajoute en 15 minutes, à une température comprise entre 20 et 30°C, 33,4 g de triéthylamine. A la suspension (suspension B) obtenue et agitée à une température voisine de 20°C pendant 50 minutes, on ajoute en 50 minutes à une température comprise entre 20 et 36°C la solution A préparée précédemment. La suspension obtenue est agitée pendant 1 heure et 40 minutes à une température voisine de 40°C puis pendant 20 minutes à une température voisine de 60°C. Après refroidissement à une température voisine de 20°C, la suspension obtenue est additionnée de 100 $cm^3$ d'eau distillée. La phase organique est séparée, lavée 3 fois par 300 $cm^3$ au total d'eau distillée, 2 fois par 300 $cm^3$ au total d'une solution aqueuse saturée de bicarbonate de sodium puis 2 fois par 300 $cm^3$ au total d'eau distillée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient 25,6 g de produit brut que l'on dissout dans 250 $cm^3$ d'acétate d'éthyle. La solution obtenue est extraite 3 fois par 300 $cm^3$ au total d'une solution aqueuse d'acide chlorhydrique 2N. Les extraits aqueux sont réunis, lavés par 250 $cm^3$ d'acétate d'éthyle et amenés à un pH voisin de 8 par addition de bicarbonate de sodium. La suspension obtenue est extraite une première fois par un mélange de 250 $cm^3$ d'ether diéthylique et de 250 $cm^3$ d'acetate d'éthyle puis 3 fois par 450 $cm^3$ au total d'acétate d'éthyle. Les extraits organiques sont réunis, lavés 2 fois par 300 $cm^3$ au total d'eau distillée, séchés sur du sulfate de magnésium anhydre, additionnés de 0,5 g de noir décolorant, filtrés, additionnés de 30 g de silice (0,020—0,045 mm), filtrés et

concentrés à sec sous pression réduite (20 mm de mercure; 2,7 kPa) à une température voisine de 60°C. On obtient ainsi 19,6 g de (+) (pyridyl-3)-3 1H,3H-pyrrolo [1,2-c] thiazolecarboxylate-7 d'éthyle sous forme d'une huile orangée. Rf = 0,5 (chromatographie sur couche mince de gel de silice; éluant: acétate d'éthyle);

$$[\alpha]_D^{20} + 115° \pm 1° \text{ (c = 1,51; diméthylformamide).}$$

Le dichloro-2,3 propionate d'éthyle peut être préparé d'après la méthode décrite dans le brevet japonais n° 81 87531 [Chem. Abstr., *95,* 203335, (1981)].

L'acide N-formyl (pyridyl-3)-2 thiazolidinecarboxylique-4-(2R, 4R) peut être obtenu de la façon suivante: A 420 $cm^3$ d'acide formique, on ajoute en 25 minutes à une température voisine de 10°C, 340 g d'anhydride acétique. La solution obtenu est agitée à une température voisine de 10°C pendant 30 minutes puis est additionnée en 50 minutes à une température voisine de 10°C de 233 g d'acide (pyridyl-3)-2 thiazolidinecarboxylique-4-(2RS, 4R). La solution obtetenue est agitée à une température voisine de 10°C pendant 30 minutes puis à une température voisine de 20°C pendant 16 heures. Le solvant est évaporé sous pression réduite (20 mm de mercure; 2,7 kPa) à une température voisine de 60°C. Le résidu obtenu est mis en suspension dans 2600 $cm^3$ d'éthanol bouillant. La suspension obtenue est refroidie à une température voisine de 4°C pendant 2 heures. Les cristaux apparus sont séparés par filtration, lavés 2 fois par 530 $cm^3$ au total d'éthanol refroidi à une température voisine de 4°C et séchés à l'air. On obtient ainsi 245 g de produit fondant à 230°C. 60 g de ce produit sont dissous dans 540 $cm^3$ d'éthanol aqueux à 50% à l'ébullition. La solution obtenue est refroidie à une température voisine de 10°C pendant 2 heures. Les cristaux apparus sont séparés par filtration, lavés 3 fois par 300 $cm^3$ au total d'éthanol et 3 fois par 450 $cm^3$ au total d'éther diéthylique puis séchés sous pression réduite (20 mm de mercure; 2,7 kPa) à une température voisine de 20°C en présence de potasse en pastilles. On obtient ainsi 48,2 g d'acide N-formyl (pyridyl-3)-2 thiazolidinecarboxylique-4-(2R, 4R) sous forme de cristaux blancs fondant à 250°C.

$$[\alpha]_D^{20} + 100° \pm 1° \text{ (c = 1,37; soude N).}$$

L'acide (pyridyl-3)-2 thiazolidinecarboxylique-4(2RS, 4R) peut être préparé selon A. BANASHEK et M.I. SHCHUKINA, J. Gen. Chem. U.S.S.R., *31,* 1374 (1961); Chem. Abstr. *55,* 24739h, (1961).

B — Deuxième procédé: 200 g d'acide (±) (pyridyl-3)-3 1H,3H-pyrrolo [1,2-c] thiazolecarboxylique-7 et 147 g de L (−)α-méthylbenzylamine sont dissous dans 1000 $cm^3$ d'isopropanol bouillant. La solution obtenue est additionnée de 0,5 g de noir décolorant et filtrée à chaud. Le filtrat est refroidi à une température voisine de 20°C pendant 16 heures. Les cristaux apparus sont séparés par filtration, lavés 3 fois par 450 $cm^3$ au total d'isopropanol refroidi à une température voisine de 4°C et 3 fois par 600 $cm^3$ au total d'éther diéthylique puis séchés sous pression réduite (20 mm de mercure; 2,7 kPa) à une température voisine de 20°C en présence de potasse en pastilles. On obtient 134,6 g de produit que l'on dissout dans 500 $cm^3$ d'isopropanol bouillant. La solution obtenue est additionnée 0,5 g de noir décolorant et filtrée à chaud. Le filtrat est refroidi à une température voisine de 20°C pendant 16 heures. Les cristaux apparus sont séparés par filtration, lavés 3 fois par 300 $cm^3$ au total d'isopropanol refroidi à une température voisine de 4°C et 2 fois par 400 $cm^3$ d'éther diéthylique puis séché sous pression réduite (20 mm de mercure; 2,7 kPa) à une température voisine de 20°C en présence de potasse en pastilles. On obtient 88,3 g de produit que l'on dissout dans 500 $cm^3$ d'isopropanol bouillant; la solution obtenue est filtrée à chaud et le filtrat est refroidi à une température voisine de 4°C pendant 16 heures. Les cristaux apparus sont séparés par filtration, lavés 2 fois par 100 $cm^3$ au total d'isopropanol refroidi à une température voisine de 4°C et 3 fois par 300 $cm^3$ au total d'éther diéthylique puis séchés sous pression réduite (20 mm de mercure; 2,7 kPa) à une température voisine de 20°C en présence de potasse en pastilles. On obtient 77,3 g de sel de L(−)α-méthylbenzylamine de l'acide (+) (pyridyl-3)-3 1H,3H-pyrrolo [1,2-c] thiazolecarboxylique-7 sous forme de cristaux crème fondant à 154°C.

$$[\alpha]_D^{20} + 110° \pm 2° \text{ (c = 1,01; eau).}$$

Ce produit est dissous dans 600 $cm^3$ d'eau distillée à une température voisine de 65°C. La solution obtenue est filtrée à chaud et refroidie à une température voisine de 10°C et est amenée à un pH voisin de 3,5 par addition d'acide chlorhydrique concentré à une température comprise entre 10 et 15°C. Les cristaux apparus sont séparés par filtration, lavés 3 fois par 600 $cm^3$ au total d'eau distillée, 2 fois par 160 $cm^3$ au total d'éthanol et 2 fois par 200 $cm^3$ au total d'éther diéthylique puis séchés sous pression réduite (20 mm de mercure; 2,7 kPa) à une température voisine de 20°C en présence de potasse en pastilles. On obtient 48 g de produit que l'on dissout dans 1000 $cm^3$ d'éthanol bouillant; la solution obtenue est additionnée de 0,5 g de noir decolorant et filtrée à chaud. Le filtrat obtenu est refroidi à une température voisine de 4°C pendant 2 heures. Les cristaux apparus sont séparés par filtration, lavés 2 fois par 60 $cm^3$ au total d'éthanol refroidi à une température voisine de 4°C puis 2 fois par 200 $cm^3$ d'éther diéthylique puis séchés sous pression réduite (20 mm de mercure: 2,7 kPa) à une température voisine de 20°C en présence de potasse en pastilles. On obtient ainsi 42,5 g d'acide (+) (pyridyl-3)-3 1H,3H-pyrrolo [1,2-c) thiazolecarboxamide-7 sous forme de cristaux jaunes fondant à 210°C.

$$[\alpha]_D^{20} + 168° \pm 2°; \text{ (c = 1,02; NaOH N).}$$

L'acide (±) (pyridyl-3)-3 1H,3H-pyrrolo [1,2-c] thiazolecarboxylique-7 peut être préparé selon la méthode décrite dans le brevet européen n°0 115 979.

Exemple 3

A une suspension de 14,3 g d'acide N-formyl (pyridyl-3)-2 thiazolidinecarboxylique-4-(2R, 4R) dans 60 $cm^3$ dans 60$cm^3$ de dichloro-1,2 éthane, on ajoute en 3 minutes, à une température comprise entre 20 et 27°C, 6,7 g de triéthylamine. La suspension obtenue est agitée à une température voisine de 20°C pendant 20 minutes et la solution obtenue est ajoutée en 1 heure, à une température comprise entre 22 et 26°C, à une solution de 12,6 g de chlorure de paratoluénesulfonyle dans 70 $cm^3$ de dichloro-1,2 éthane. On obtient une suspension fine (suspenson A). Par ailleurs, à une solution de 19,3 g de N-(benzoyl-3 phényl) dichloro-2,3 propionamide dans 100 $cm^3$ de dichloro-1,2 éthane, on ajoute en 15 minutes, à une température comprise entre 20 et 30°C, 20 g de triethylamine. On obitent ainsi une suspension (suspension B) que l'on agite à une tempéature voisine de 20°C pendant 16 heures. A cette suspension B on ajoute en 10 minutes à une température comprise entre 22 et 40°C la suspension A préparée précédemment. La nouvelle suspension obtenue est chauffée à une température voisine de 86°C pendant 1 heure. Après refroidissement à une température voisine de 20°C, le mélange réactionnel est lavé 3 fois par 600 $cm^3$ au total d'eau distillée. La phase organique est séparée, séchée sur du sulfate de magnésium anhydre, additionnée de 0,5 g der noir décolorant, filtrée et concentrée à sec sous pression réduite (20 mm de mercure; 2,7 kPa) à une température voisine de 60°C. On obtient 29,3 g de produit que l'on dissout dans 500 $cm^3$ d'acétate d'éthyle; la solution obtenue est additionnée de 0,5 g de noir decolorant et de 30 g de silice (0,020—0,045 mm), filtrée et concentrée à sec sous pression réduite (20 mm de mercure; 2.7 kPa) à une température voisine de 60°C. On obtient 25,5 g de produit brut que l'on dissout et dans 130 $cm^3$ d'un mélange bouillant d'ethanol et d'eau (85—15 en volumes). La solution obtenue est additionnée de 0,5 g de noir décolorant et filtrée à chaud. Le filtrat est refroidi à une température voisine de 20°C pendant 16 heures. Les cristaux apparus sont séparés par filtration, lavés 2 fois par 60 $cm^3$ au total d'un mélange d'éthanol et d'eau (85—15 en volumes) et 3 fois par 150 $cm^3$ au total d'éther diéthylique puis séchés sous pression réduite (20 mm de mercure; 2.7 kPa) à une température voisine de 20°C en présence de potasse en pastilles. On obtient ainsi 13,75 g de (+) (N-benzoyl-3 phényl) (pyridyl-3)-3 1H,3H-pyrrolo [1,2-c] thiazolecarboxamide-7 à l'état d'hydrate sous forme de cristaux crème fondant à 110°C.

$$[\alpha]_D^{20} + 88,2° \pm 1° \ (c = 1,02;\ \text{diméthylformamide}).$$

L'acide N-formyl (pyridyl-3)-2 thiazolidinecarboxylique-4-(2R,4R) peut être préparé comme indiqué à l'exemple 2.

Le (N-benzoyl-3 phényl) dichloro-2,3 propionamide peut être preparé de la façon suivante: A une solution de 29,6 g d'amino-3 benzophénone dans 400 $cm^3$ de toluène, on ajoute en 20 minutes à une température comprise entre 60 et 105°C, une solution de 24,2 g de chlorure de dichloro-2,3 propionyle dans 80 $cm^3$ de toluène. La solution obtenue est chauffée à une température voisine de 110°C pendant 3 heures puis est agitée à une température voisine de 20°C pendant 16 heures. Le solvant est évaporé sous pression réduite (20 mm de mercure; 2,7 kPa) à une température voisine de 60°C. Le résidu est dissous dans 250 $cm^3$ d'oxyde d'isopropyle bouillant. La solution obtenue est refroidie à une température voisine de 20°C pendant 16 heures. Les cristaux apparus sont séparés par filtration, lavés 3 fois par 300 $cm^3$ au total d'oxyde d'isopropyle et séchés sous pression réduite (20 mm de mercure; 2,7 kPa) à une température voisine de 20°C en présence de potasse en pastilles. On obtient ainsi 38,9 g de N-(benzoyl-3 phényl) dichloro-2,3 propionamide sous forme de cristaux crème fondant à 92°C.

L'amino-3 benzophénone peut être préparé d'après R. GEIGY et W. KOENIGS, Ber., *18,* 2400 (1885).

Exemple 4

A une solution de 6,6 g de (chloro-4-phénoxy)-3 aniline et de 6,1 g de triéthylamine dans 200 $cm^3$ de dioxanne chauffée à une température voisine de 60°C on ajoute en 25 minutes à une température voisine de 60°C, 9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 5 heures puis est agitée à une tempefrature voisine de 20°C pendant 16 heures. Le solvant est èvaporé sous pression réduite (20 mm de mercure; 2,7 kPa) à une température voisine de 65°C. Le résidu rest dissous dans 400 $cm^3$ de chlorure de méthylène et la solution obtenue est lavée 2 fois par 200 $cm^3$ au total d'eau distillée, 2 fois par 200 $cm^3$ au total d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 13 g de produit que l'on chromatographie sur une colonne de 3 cm de diamètre contenant 120 g de silice (0,063—0,2 mm). On élue par des mélanges de chlorure de méthylène et de méthanol en recueillant des fractions de 300 $cm^3$. Les 5 premières fractions provenant de l'élution par du chlorure de méthylène pur sont éliminées. Les 9 fractions suivantes provenant de l'elution par un mélange de chlorure de méthylène et de méthanol (99—1 en volumes) sont réunies et concentrées à sec sous pression réduite (20 mm der mercure; 2,7 kPa) à une température voisine de 50°C. On obtient ainsi 9,4 g de produit brut que l'on dissout et dans 100 $cm^3$ d'isopropanol bouillant. La solution obtenue est additionnée de 0,5 g de noir décolorant et

filtrée à chaud. Le filtrat obtenu est refroidi à une température voisine de 4°C pendant 16 heures. Les cristaux apparus sont séparés par filtration, lavés 2 fois par 20 $cm^3$ au total d'isopropanol refroidi à une température voisine de 4°C et 2 fois par 40 $cm^3$ au total d'éther diéthylique puis séchés sous pression réduite (20 mm de mercure; 2,7 kPa) à une température voisine de 20°C en présence de potasse en pastilles. On obtient ainsi 6,5 g de N-[(chloro-4-phénoxy)-3-phényl] (pyridyl-3)-3 1H,3H-pyrrolo [1,2-c] thiazolecarboxamide-7 sous forme de cristaux crème fondant à 140°C.

La (chloro-4-phénoxy)-3 aniline peut être préparée d'après K. IKAWA, J. Pharm. Soc. Jap., *79,* 269 (1959).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 5

A une solution de 6 g de (méthyl-4-phénoxy)-3 aniline et de 6,1 g de triéthylamine dans 150 $cm^3$ de dioxanne chauffée à une température voisine de 60°C, on ajoute en 35 minutes à une température comprise entre 61 et 65°C, 9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 60°C. Le résidu est dissous dans 400 $cm^3$ de chlorure de méthylène. La solution obtenue est lavée 2 fois par 200 $cm^3$ au total d'eau distillée, 2 fois par 200 $cm^3$ au total d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 7 g de produit brut que l'on dissout dans 100 $cm^3$ d'acétonitrile bouillant. La solution obtenue est additionée de 0,5 g de noir décolorant et filtrée à chaud. Le filtrat est refoidi à une température voisine de 4°C pendant 1 heure. Les cristaux apparus sont séparés par filtration, lavés 2 fois par 20 $cm^3$ au total acétonitrile refroidi à une température voisine de 4°C et 2 fois par 20 $cm^3$ au total d'éther diéthylique puis séchés sous pression réduite (20 mm de mercure; 2,7 kPa) à une température voisine de 20°C en présence de potasse en pastilles. On obtient ainsi 3,6 g de N-[(méthyl-2 phénoxy)-3 phényl] (pyridyl-3)-3 1H,3H-pyrrolo [1,2-c] thiazolecarboxamide-7 sous forme de cristaux blancs fondant à 180°C.

La (méthyl-2-phénoxy)-3 aniline peut être preparée selon la méthode décrite dans le brevet néerlandais n°66/2994.

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 6

A une solution de 6 g de (méthyl-3-phénoxy)-3 aniline et de 6,1 g de triéthylamine dans 200 $cm^3$ de dioxanne chauffée à une température voisine de 60°C, on ajoute en 15 minutes à une température comprise entre 60 et 64°C, 9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 4 heures et 30 minutes puis est agitée à une température voisine de 20°C pendant 16 heures. Le solvant est évaporé sous pression réduite (20 mm de mercure; 2,7 kPa) à une température voisine de 60°C. Le résidu obtenu est dissous dans 350 $cm^3$ de chlorure de méthylène et la solution obtenue est lavée 2 fois par 200 $cm^3$ au total d'eau distillée, 2 fois par 200 $cm^3$ au total d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 13 g de produit brut. Ce produit est dissous dans 50 $cm^3$ d'acétonitrile bouillant. La solution obtenue est additionnée de 0,5 g de noir décolorant et filtrée à chaud. Le filtrat obtenue est refroidi à une température voisine de 4°C pendant 2 heures. Les cristaux apparus sont séparés par filtration, lavés 2 fois par 10 $cm^3$ au total d'acétonitrile refroidi à une température voisine de 4°C et séchés sous pression réduite (20 mm de mercure; 2,7 kPa) à une température voisine de 20°C en présence de potasse en pastilles. On obtient ainsi 6,6 g de produit fondant à 148°C que l'on dissout dans 100 $cm^3$ d'isopropanol bouillant. La solution obtenue est refroidie à une température voisine de 4°C pendant 2 heures. Les cristaux apparus sont séparés par filtration, lavés 2 fois par 20 $cm^3$ d'isopropanol refroidi à une température voisine de 4°C puis 2 fois par 40 $cm^3$ au total d'éther diéthylique et séchés sous pression réduite (20 mm de mercure; 2,7 kPa) à une température voisine de 20°C en présence de potasse en pastilles. On obtient ainsi 5,5 g de N-[(méthyl-3 phénoxy)-3 phényl] (pyridyl-3)-3 1H,3H-pyrrolo [1,2-c] thiazolecarboxamide-7 sous forme de cristaux crème fondant à 149°C.

La (méthyl-3 phénoxy)-3 aniline peut être préparée selon K. IKAWA, J. Pharm. Soc. Jap., *75,* 457 (1955); Chem. Abstr., *50,* 2480 (1956).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 7

A une solution de 8 g de (méthyl-4 phénoxy)-3 aniline et de 8,1 g de triéthylamine dans 200 $cm^3$ de dioxanne chauffée à une température voisine de 60°C, on ajoute en 5 minutes à une température comprise

entre 60 et 70°C, 12 g de chlorhydrate de chloroformyl-7 (pyridyl-3)-3 1H,3H-pyrrolo [1,2-c] thiazole. La suspension obtenue est chauffée sous agitation à une température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène et la solution obtenue est lavée 2 fois par 500 $cm^3$ au total d'eau distillée, 2 fois par 300 $cm^3$ au total d'une solution aqueuse de soude 2N et 2 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 16,8 g de produit que l'on chromatographie sur une colonne de 8 cm de diamètre contenant 640 g de silice (0,02—0,045 mm). On élue par de l'acétate d'ethyle sous une pression de 0,4 bar (40 kPa) en recueillant des fractions de 500 $cm^3$. Les 5 premières fractions sont éliminées. Les 4 fractions suivantes sont réunies et concentreés à sec sous pression réduite (20 mm de mercure; 2,7 kPa) à une température voisine de 60°C. On obtient ainsi 13 g de produit brut que l'on dissout dans 40 $cm^3$ d'un mélange bouillant de cyclohexane et d'acétate d'éthyle (50—50 en volumes). La solution obtenue est additionnée de 0,5 g de noir décolorant et filtrée à chaud. Le filtrat obtenu est refroidi à une tempérture voisine de 4°C pendant 16 heures. Les cristaux apparus sont séparés par filtration, lavés 2 fois par 50 $cm^3$ au total d'un melange de cyclohexane et d'acétate d'éthyle (50—50 en volumes) refroidi à une température voisine de 4°C et 2 fois par 50 $cm^3$ au total d'éther diéthylique puis séchés sous pression réduite (20 mm de mercure; 2,7 kPa) à une température voisine de 20°C en présence de potasse en pastilles. On obtient ainsi 8,9 g de N-[(méthyl-4 phénoxy)-3 phényl] (pyridyl-3)-3 1H,3H-pyrrolo [1,2-c] thiazolecarboxamide-7 sous forme de cristaux blancs fondant à 110°C.

La (méthyl-4-phénoxy)-3 aniline peut être préparée selon la méthode décrite dans la brevet néerlandais n° 66/2994.

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 8

A une solution de 7,1 g de (méthoxy-2-phénoxy)-3 aniline et de 6,7 g de triéthylamine dans 160 $cm^3$ de dioxanne chauffée à une température voisine de 60°C, on ajoute en 30 minutes à une température comprise entre 61 et 65°C, 10 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 60°C. Le résidu est dissous dans 400 $cm^3$ de chlorure de méthylène. La solution obtenue est lavée par 100 $cm^3$ d'eau distillé, 2 fois par 200 $cm^3$ au total d'une solution aqueuse de soude N, et 6 fois par 600 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 7,8 g de produit brut que l'on dissout dans 50 $cm^3$ d'acétonitrile bouillant. La solution obtenue est additionée de 0,5 g de noir décolorant et filtrée à chaud. Le filtrat est refroidi à une tempérture voisine de 4°C pendant 1 heure. Les cristaux apparus sont séparés par filtration, lavés 2 fois par 15 $cm^3$ au total d'acétonitrile refroidi à une température voisine de 4°C et 2 fois par 30 $cm^3$ au total d'éther diéthylique puis seches sous pression réduite (20 mm de mercure; 2,7 kPa) à une température voisine de 20°C en présence de potasse en pastilles. On obtient ainsi 3,9 g de N-[(méthoxy-2 phénoxy)-3 phényl] (pyridyl-3)-3 1H,3H-pyrrolo [1,2-c] thiazolecarboxamide-7 sous forme de cristaux blancs fondant à 163°C.

La (méthoxy-2 phénoxy)-3 aniline est préparée selon K. IKAWA, J. Pharm. Soc. Jap., *79,* 1493 (1959); Chem. Abstr., *54,* 10922 (1960).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 9

A une solution de 6,45 g de (méthoxy-3-phénoxy)-3 aniline et de 6,1 g de triéthylamine dans 200 $cm^3$ de dioxanne chauffée à une température voisine de 60°C, on ajoute en 15 minutes à une température comprise entre 60 et 65°C, 9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 4 heures et 30 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 350 $cm^3$ de chlorure de méthylène. La solution obtenue est lavée 2 fois par 200 $cm^3$ au total d'eau distilleé, 2 fois par 200 $cm^3$ au total d'une solution aqueuse de soude N, et 5 fois par 750 $cm^3$ au total d'eau distillé puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 12 g de produit brut que l'on dissout dans 50 $cm^3$ d'acétonitrile bouillant. La solution obtenue est additionnée de 0,5 g de noir décolorant et filtrée à chaud. Le filtrat est refroidi à une température voisine de 4°C pendant 2 heures. Les cristaux apparus sont séparés par filtration, lavés 2 fois par 10 $cm^3$ au total d'acétonitrile refroidi à une température voisine de 4°C puis séchés sous pression réduite (20 mm de mercure; 2,7 kPa) à une température voisine de 20°C en présence de potasse en pastilles. On obtient ainsi

4,5 g de N-[(méthoxy-3 phénoxy)-3-phényl] (pyridyl-3)-3 1H,3H-pyrrolo [1,2-c] thiazolecarboxamide-7 sous forme de cristaux crème fondant à 130°C.

La (méthoxy-3 phénoxy)-3 aniline peut être préparée selon K. IKAWA, J. Pharm. Soc. Jap., *79,* 1493 (1959).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 10

A une solution de 6,45 g de (méthoxy-4 phénoxy)-3 aniline et de 6,1 g de triéthylamine dans 200 $cm^3$ de dioxanne chauffée à une température voisine de 60°C, on ajoute en 15 minutes à une température voisine de 60°C, 9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 5 heures puis est agité à une température voisine de 20°C pendant 12 heures. Le solvant est évaporé sous pression réduite (20 mm de mercure; 2,7 kPa) à une température voisine de 65°C. Le résidu est dissous dans 400 $cm^3$ de chlorure de méthylène et la solution obtenue est lavée 2 fois par 200 $cm^3$ au total d'eau distilleé, 2 fois par 200 $cm^3$ au total d'une solution aqueuse de soude N et 5 fois par 500 $cm^3$ au total d'eau distillée puis séchés sur du sulfate de magnésium anhydre, additionnée de 0,5 g de noir décolorant, filtrée et concertrée à sec sous pression réduite (20 mm de mercure; 2,7 kPa) à une température voisine de 50°C. On obtient ainsi 12,5 g de produit que l'on chromatographie sur une colonne de 3 $cm^3$ de diamètre contenant 120 g de silice (0,063—0,2 mm). On élue par des mélanges de chlorure de méthylène et de méthanol en recueillant des fractions de 300 $cm^3$. Les 5 premières fractions provenant de l'élution par du chlorure de méthylène par sont éliminées. Les 10 fractions suivantes provenant de l'élution par un mélange de chlorure de méthylène et de méthanol (99—1 en volumes) sont réunies et concentrées à sec sous pression réduite (20 mm de mercure; 2,7 kPa) à une température voisine de 50°C. On obtient ainsi 9,2 g de produit brut que l'on dissout dans 85 $cm^3$ d'isopropanol bouillant. La solution obtenue est additionnée de 0,5 g de noir décolorant et filtrée à chaud. Le filtrat obtenue est refroidi à une température voisine de 4°C pendant 16 heures. Les cristaux apparus sont séparés par filtration, lavés 2 fois par 20 $cm^3$ d'isopropanol refroidi à une température voisine de 4°C et 2 fois par 40 $cm^3$ au total d'éther diéthylique puis séchés sous pression réduite (20 mm de mercure; 2,7 kPa) à une température voisine de 20°C en présence de potasse en pastilles. On obtient ainsi 5 g de N-[(méthoxy-4 phénoxy)-3 phényl] (pyridyl-3)-3 1H,3H-pyrrolo [1,2-c] thiazolecarboxamide-7 sous forme de cristaux crème fondant à 120°C.

La (méthoxy-4 phénoxy)-3 aniline peut être préparée selon K. IKAWA, J. Pharm., Soc., Jap., *79,* 1493 (1959).

Le chlorhydrate de chloroformyl-7 (pyridyl-3)-3 1H,3H-pyrrolo [1,2-c) thiazole est préparé selon la méthode décrite dans le brevet européen n° 0 115 979.

Exemple 11

A une solution de 6 g de méthoxy-2 phénoxy-5 aniline et de 5,7 g de triéthylamine dans 100 $cm^3$ de dioxanne chauffée à une température voisine de 60°C, on ajoute en 30 minutes à une température comprise entre 60 et 65°C, 8,4 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 60°C. Le résidu est repris par un mélange de 150 $cm^3$ d'une solution aqueuse de soude N et de 350 $cm^3$ de chlorure de méthylène. La phase organique est séparée, lavée 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 9 g de produit brut que l'on met en suspension dans 30 $cm^3$ d'un mélange de cyclohexane et d'acétate d'éthyle (50—50 en volumes). Les cristaux apparus sont séparés par filtration, lavés 2 fois par 10 $cm^3$ au total d'un mélange de cyclohexane et d'acétate d'éthyle (50—50 en volumes) puis séchés sous pression réduite (20 mm de mercure; 2,7 kPa) à une température voisine de 20°C en présence de potasse en pastilles. On obtient ainsi 6,6 g de produit fondant à 148°C que l'on dissout dans 50 $cm^3$ d'acétonitrile bouillant; la solution obtenue est additionée de 0,5 g de noir décolorant et filtrée à chaud. Le filtrat est refroidi à une température voisine de 4°C pendant 16 heures. Les cristaux apparus sont séparés par filtration, lavés 2 fois par 20 $cm^3$ au total d'acétonitrile refroidi à une température voisine de 4°C puis séchés sous pression réduite (20 mm de mercure; 2,7 kPa) à une température voisine de 20°C en présence de potasse en pastilles. On obtient ainsi 5 g de N-(méthoxy-2 phénoxy-5 phényl) (pyridyl-3)-3 1H,3H-pyrrolo [1,2-c] thiazolecarboxamide-7 sous forme de cristaux blancs fondant à 158°C.

La méthoxy-2-phénoxy-5 aniline peut être obtenue selon G. SCHIEMANN et W. WINKELMULLER, J. Prakt. Chem., *135,* 101 (1932).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 12

A une solution de 7,4 g de (pyridyl-2-oxy)-3 aniline et de 8,1 g de triéthylamine dans 200 $cm^3$ de

dioxanne chauffée à une température voisine de 60°C, on ajoute en 5 minutes à une température comprise entre 60 et 68°C, 12 g de chlorhydrate de chloroformyl-7 (pyridyl-3)-3 1H,3H-pyrrolo [1,2-c] thiazole. La suspension obtenue est chauffée sous agitation à une température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 500 $cm^3$ au total d'eau distillée, 2 fois par 300 $cm^3$ au total d'une solution aqueuse de soude N et 2 fois par 500 $cm^3$ au total d'eau distillée puis séchée sur du sulfate de magnésium anhydre, additionnée de 0,5 g de noir décolorant, filtrée et concentrée à sec sous pression reduite (20 mm de mercure; 2,7 kPa) à une température voisine de 60°C. On obtient ainsi 15,8 g de produit brut que l'on dissout dans 100 $cm^3$ d'acétonitrile bouillant. La solution obtenue est additionnée de 0,5 g de noir décolorant et filtrée à chaud. Le filtrat est refroidi à une température voisine de 20°C pendant 3 heures. Les cristaux apparus sont séparés par filtration, lavés 3 fois par 45 $cm^3$ au total d'acétonitrile et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 8,5 g de N-[(pyridyl-2 oxy)-3 phényl] (pyridyl-3)-3 1H,3H-pyrrolo [1,2-c] thiazolecarboxamide-7 sous forme de cristaux crème fondant à 132°C.

La (pyridyl-2 oxy)-3 aniline peut être préparée selon la méthode décrite dans la brevet allemand n° 3 139 457.

Le chlorhydrate de chloroformyl-7 (pyridyl-3)-3 1H,3H-pyrrolo [1,2-c] thiazole est préparé selon la méthode décrite dans le de brevet européen n° 0 115 979.

Exemple 13

A une solution de 3,9 g de (pyridyl-3 oxy)-3 aniline et de 4,05 g de triéthylamine dans 150 $cm^3$ de dioxanne chauffée à une température voisine de 65°C, on ajoute en 5 minutes à une température comprise entre 65 et 72°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures et 30 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau distillée, 2 fois par 300 $cm^3$ au total d'une solution aqueuse de soude N, et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 8,2 g de produit brut que l'on dissout dans 50 $cm^3$ d'acétonitrile bouillant. La solution obtenue est additionnée de 0,5 g de noir décolorant et filtrée à chaud. Le filtrat est refroidi à une température voisine de 20°C pendant 2 heures. Les cristaux apparus sont séparés par filtration, lavés 3 fois par 30 $cm^3$ au total d'acétonitrile et 3 fois par 90 $cm^3$ au total d'ether diéthylique puis séchés sous pression réduite (20 mm der mercure; 2,7 kPa) à une température voisine de 20°C en présence de potasse en pastilles. On obtient ainsi 3,4 g de N-[(pyridyl-3 oxy)-3 phényl] (pyridyl-3)-3 1H,3H-pyrrolo [1,2-c] thiazolecarboxamide-7 sous forme de cristaux crème fondant à 154°C.

Le chlorhydrate de chloroforyml-7 (pyridyl-3)-3 1H,3H-pyrrolo [1,2-c] thiazole est préparé selon la méthode décrite dans le brevet européen n° 0 115 979.

La (pyridyl-3 oxy)-3 aniline est preparée de la façon suivante: A une suspension de 16,2 g de (pyridyl-3 oxy)-3 nitrobenzène et de 37,5 g de fer en poudre dans 40 $cm^3$ d'eau distilliée chauffée à une température voisine de 90°C, on ajoute à une température comprise entre 90 et 98°C, 0,4 g der chlorure ferrique. La suspension obtenue est chauffée à une température voisine de 98°C penant 1 heure et 15 minutes puis est agitée à une température voisine de 20°C pendant 16 heures, additionée de 550 $cm^3$ de chlorure de méthylène et de 75 $cm^3$ d'eau distillée et filtrée. La phase organique est séparée, séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 13,1 g d'huile brute que l'on chromatographie sur une colonne de 6 cm de diamètre contenant 450 g de silice (0,02—0,045 mm). On élue par des mélanges d'acétate d'ethyle et de cyclohexane sous une pression de 0,4 bar (40 kPa) en recueillant des fractions de 150 $cm^3$. Les 15 premières fractions provenant de l'élution par un mélange d'acétate d'éthyle et de cyclohexane (50—50 en volumes) sont éliminées. Les 5 fractions suivantes provenant de l'élution par un mélange d'acétate d'éthyle et cyclohexane (50—50 en volumes) et les 5 fractions suivantes provenant de l'élution par de l'acétate d'éthyle pur sont réunies et concentrées à sec sous pression réduite (20 mm de mercure; 2,7 kPa) à une température voisine de 60°C. On obtient ainsi 8,4 g de (pyridyl-3 oxy)-3-aniline sous forme d'un liquide jaune orangé [Rf = 0,25; chromatographie sur couche mince de gel de silice; éluant: cyclohexane-acétate d'éthyle (50—50 en volumes)].

Le (pyridyl-3 oxy)-3 nitrobenzène est préparé de la façon suivante: Une solution de 47,5 g d'hydroxy-3 pyridine et de 33 g de potasse en pastilles dans 400 $cm^3$ d'éthanol est chauffée à une température voisine de 80°C pendant 1 heure. Le solvant est évaporé sous pression réduite (20 mm de mercure; 2,7 kPa) à une température voisine de 70°C et le résidu est dissous dans 350 $cm^3$ de diméthylsulfoxyde. La solution est additonnée de 101 g de bromo-3 nitrobenzène et de 0,1 g de poudre de cuivre puis est chauffée sous courant d'azote sec à une température voisine de 160°C pendant 1 heure. Le mélange réactionnel obtenue est refroidi à une température voisine de 20°C, additionné de 2500 $cm^3$ d'eau distillée et de 500 $cm^3$

de chlorure de méthylène. La phase organique est séparée et la phase aqueuse est extraite 2 fois par 1000 $cm^3$ au total de chlorure de méthylène. Les extraits organiques sont réunis, lavés 3 fois par 1500 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 60°C. L'huile brute obtenue (77 g) est distillée sous pression réduite. On obtient ainsi 41,8 g de (pyridyl-3 oxy)-3 nitrobenzène sous forme de liquide orange bouillant à 165—175°C sous 0,1 mm de mercure (13, 5 Pa).

Exemple 14

A une solution de 15,7 g d'amino-3 benzophénone et de 16,1 g de triéthylamine dans 400 $cm^3$ de dioxanne chauffée à une température voisine de 65°C, on ajoute en 15 minutes à une température voisine de 65°C, 24 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 60°C. Le résidu est dissous dans 500 $cm^3$ de chlorure de méthylène est la solution obtenue est lavée 2 fois par 200 $cm^3$ au total d'eau distillée, 4 fois par 800 $cm^3$ au total d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. La produit obtenu est chromatographié sur une colonne de 8,5 cm de diamètre contenant 1 kg der silice (0,02—0,045 mm). On élue par des mélanges de cyclohexane et d'acétate d'éthyle sous une pression de 0,4 bar (40 kPa) en recueillant des fractions de 500 $cm^3$. Les 10 premières fractions provenant de l'élution par un mélange d'acétate d'éthyle et de cyclohexane (60—40 en volumes) et les 5 fractions suivantes provenant de l'élution par un mélange d'acétate d'éthyle et de cyclohexane (80—20 en volumes) sont éliminées. Les 10 fractions suivantes provenant de l'élution par un mélange d'acétate d'éthyle et de cyclohexane (80—20 en volumes) et les 2 fractions suivantes provenant de l'élution par un mélange d'acétate d'éthyle et de cyclohexane (90—10 en volumes) sont réunies et concentrées à sec sous pression réduite (20 mm de mercure; 2,7 kPa) à une température voisine de 50°C. On obtient ainsi 26,5 g de produit brut que l'on dissout dans 150 $cm^3$ d'acétonitrile bouillant. La solution obtenue est additionnée de 0,5 g de noir décolorant et filtrée à chaud. Le filtrat obtenu est refroidi à une température voisine de 45°C pendant 16 heures. Les cristaux apparus sont séparés par filtration, lavés 3 fois par 90 $cm^3$ au total d'acétonitrile refroidi à une température voisine de 4°C et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 14 g de N-(benzoyl-3 phényl) (pyridyl-3)-3 1H,3H-pyrrolo [1,2-c] thiazolecarboxamide-7 sous forme de cristaux crème fondant à 154°C.

L'amino-3 benzophénone peut être préparée selon R. GEIGY et W. KOENIGS, Ber., *18,* 2400 (1885).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 15

A une solution de 4,6 g d'amino-3 chloro-4' benzophénone et de 4,05 g de triéthylamine dans 150 $cm^3$ de dioxanne chauffée à une température voisine de 66°C, on ajoute en 5 minutes à une température comprise entre 66 et 72°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 8 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau distillée, 2 fois par 300 $cm^3$ au total d'une solution aqueuse de soude 2N, et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 9,8 g de produit brut que l'on dissout dans 250 $cm^3$ d'acétonitrile bouillant. La solution obtenue est additionée de 0,5 g de noir décolorant et filtrée à chaud. Le filtrat est refroidi à une température voisine de 10°C pendant 3 heures. Les cristaux apparus sont séparés par filtration, lavés 3 fois par 45 $cm^3$ au total d'acétonitrile et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 3,2 g de N-[(chloro-4 benzoyl)-3 phenyl] (pyridyl-3)-3 1H,3H-pyrrolo [1,2-c] thiazolecarboxamide-7 sous forme de cristaux jaune pâle fondant à 176°C.

L'amino-3 chloro-4'benzophénone peut être obtenue selon F. E. KING, T. J. KING ET I. H. M. MUIR, J. Chem. Soc., 5, (1946).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 16

A une solution de 4,25 g d'amino-3 méthyl-3' benzophénone et de 4,05 g de triéthylamine dans 150 $cm^3$ de dioxanne chauffée à une température voisine de 64°C, on ajoute en 5 minutes à une température comprise entre 64 et 70°C, 6 g de chlorhydrate de chloroformyl-7 (pyridyl-3)-3 1H,3H-pyrrolo [1,2-c] thiazole.

La suspension obtenue est chauffée sous agitation à une température voisine de 100°C pendant 8 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau distillée, 2 fois par 300 $cm^3$ au total d'une solution aqueuse de soude N et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 9 g de produit brut que l'on dissout dans 720 $cm^3$ d'acétonitrile bouillant. La solution obtenue est additionnée de 0,5 g de noir décolorant et filtrée à chaud. Le filtrat est refroidi à une température voisine de 10°C pendant 3 heures. Les cristaux apparus sont séparés par filtration, lavés 3 fois par 30 $cm^3$ au total d'acétonitrile refroidi à une température voisine de 4°C et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 2,4 g de N-[(méthyl-3 benzoyl)-3 phényl] (pyridyl-3)-3 1H,3H-pyrrolo [1,2-c] thiazolecarboxamide-7 sous forme de cristaux beiges fondant à 164°C.

Le chlorhydrate de chloroformyl-7 (pyridyl-3)-3 1H,3H-pyrrolo [1,2-c] thiazole est préparé selon la méthode décrite dans le brevet européen n° 0 115 979.

L'amino-3 méthyl-3' benzophénone peut être préparée de la façon suivante: A une suspension de 6,8 g de méthylé-3' nitro-3 benzophénone dans un mélange de 80 $cm^3$ d'éthanol et de 30 $cm^3$ d'acide chlorhydrique concentré, on ajoute en 10 minutes à une température comprise entre 40 et 80°C, 19 g de chlorure stanneux à l'état de dihydrate. La solution obtenue est chauffée à une température voisine de 85°C pendant 3 heures puis est agitée à une température voisine de 20°C pendant 16 heures. Le solvant est évaporé sous pression réduite (20 mm de mercure; 2,7 kPa) à une température voisine de 60°C. Le résidu est mis en suspension dans 300 $cm^3$ d'eau distillée. La suspension obtenue est additionnée sous agitation à une température voisine de 20°C de 100 $cm^3$ d'une solution aqueuse de soude 10N et est extraite 3 fois par 550 $cm^3$ au total d'éther diéthylique. Les extraits éthérés sont réunis, lavés 2 fois par 300 $cm^3$ au total d'eau distillée puis séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient ainsi 5,7 g d'amino-3 méthyl-3' benzophénone sous forme de cristaux jaunes fondant à 110°C.

La méthyl-3' nitro-3 benzophénone peut être obtenue d'après K. DEY, C. EABORN et D.R.M. WALTON, Organometal Chem. Syn., *1*, 151 (1971).

Exemple 17

A une solution de 4,55 g d'amino-3 méthoxy-4' benzophénone et de 4,05 g de triéthylamine dans 100 $cm^3$ de dioxanne chauffée à une température voisine de 62°C, on ajoute en 15 minutes à une température comprise entre 62 et 65°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 5 heures puis est agitée à une température voisine de 20°C pendant 16 heures. Le solvant est évaporé sous pression réduite (20 mm de mercure; 2,7 kPa) à une température voisine de 60°C. Le résidu est dissous dans 200 $cm^3$ de chlorure de méthylène et la solution obtenue est lavée 2 fois par 200 $cm^3$ au total d'eau distillée, 2 fois par 200 $cm^3$ au total d'une solution aqueuse de soude N et 3 fois par 300 $cm^3$ au total d'eau distillé puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 6,7 g de produit que l'on chromatographie sur une colonne de 5,5 cm de diamètre contenant 500 g de silice (0,02—0,045 mm). On élue par de l'acétate d'éthyle sous une pression de 0,4 bar (40 kPa) en recueillant des fractions de 250 $cm^3$. Les 9 premières fractions sont éliminées. Les 6 fractions suivantes sont réunies et concentrées à sec sous pression réduite (20 mm de mercure; 2,7 kPa) à une température voisine de 50°C. On obtient ainsi 5 g de produit brut. Ce produit est dissous dans 40 $cm^3$ d'acétonitrile bouillant. La solution obtenue est additionnée de 0,5 g de noir décolorant et filtrée à chaud. Le filtrat obtenu est refroidi à une température voisine de 4°C pendant 2 heures. Les cristaux apparus sont séparés par filtration, lavés 2 fois par 15 $cm^3$ au total d'acétonitrile refroidi à une température voisine de 4°C et 2 fois par 20 $cm^3$ au total d'ether diéthylique puis séchés sous pression réduite (20 mm de mercure; 2,7 kPa) à une température voisine de 20°C en présence de potasse en pastilles. On obtient ainsi 3,9 g de N-[(méthoxy-4 benzoyl)-3 phényl] (pyridyl-3)-3 1H,3H-pyrrolo [1,2-c] thiazolecarboxamide-7 sous forme de cristaux crème fondant à 140°C.

L'amino-3 méthoxy-4' benzophénone peut être préparée selon H. OELSCHLAGER, Arzneim. Forsch., *8*, 532 (1958).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 18

A une solution de 5,75 g d'(amino-3 benzoyl)-3 pyridine et de 4,65 g de triéthylamine dans 120 $cm^3$ de dioxanne chauffée à une température voisine de 50°C, on ajoute en 25 minutes à une température comprise entre 50 et 85°C, 6,9 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression

réduite (20 mm de mercure; 2,7 kPa) à une température voisine de 60°C. Le résidu est dissous dans 350 $cm^3$ de chlorure de méthylène et la solution obtenue est lavée 2 fois par 200 $cm^3$ au total d'eau distillée, 2 fois par 200 $cm^3$ au total d'une solution aqueuse de soude 2N et 3 fois par 450 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On chromatographie le produit obtenu sur une colonne de 8 cm de diamètre contenant 500 g de silice (0,02—0,045 mm). On élue par des mélanges d'acétate d'éthyle et de méthanol sous une pression de 0,4 bar (40 kPa) en recueillant des fractions der 500 $cm^3$. Les 19 premières fractions provenant de l'élution par de l'acétate d'éthyle pur sont éliminées. Les 2 fractions suivantes provenant de l'élution par de l'acétate d'éthyle pur et les 2 fractions suivantes provenant de l'élution par un mélange d'acétate d'éthyle et de méthanol (90—10 en volumes) sont réunies et concentrées à sec sous pression réduite (20 mm de mercure; 2,7 kPa) à une température voisine de 50°C. On obtient ainsi 8 g de produit brut. Ce produit est dissous dans 50 $cm^3$ d'acétonitrile bouillant. La solution obtenue est additionnée de 0,5 g de noir décolorant et filtrée à chaud. Le filtrat obtenu est refroidi à une température voisine de 4°C pendant 1 heure. Les cristaux apparus sont séparés par filtration, lavés 2 fois par 20 $cm^3$ au total d'acétonitrile refroidi à une température voisine de 4°C et 2 fois par 40 $cm^3$ au total d'éther diéthylique puis séchés sous pression réduite (20 mm de mercure; 2,7 kPa) à une température voisine de 20°C en présence de potasse en pastilles. On obtient ainsi 4,9 g de N-(nicotinoyl-3 phényl) (pyridyl-3)-3 1H,3H-pyrrolo [1,2-c] thiazolecarboxamide-7 sous forme de cristaux crème fondant à 160°C.

L'(amino-3 benzoyl)-3 pyridine peut être obtenue selon T. HOGBERG, B. ULFF, A. L. RENYI et S. B. RÖSS, J. Med. Chem., *24,* 1499 (1981).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 19

A une solution de 3,95 g d'(amino-3 benzoyl)-2 pyridine et de 4,05 g de triéthylamine dans 100 $cm^3$ de dioxanne chauffée à une température voisine de 60°C, on ajoute en 15 minutes à une température comprise entre 60 et 67°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 5 heures puis est agitée à une température voisine de 20°C pendant 16 heures. Le solvant est évaporé sous pression réduite (20 mm de mercure; 2,7 kPa) à une température voisine de 60°C. Le résidu est dissous dans 200 $cm^3$ de chlorure de méthylène et la solution obtenue est lavée 2 fois par 200 $cm^3$ au total d'eau distillée, 2 fois par 200 $cm^3$ au total d'une solution aqueuse de soude N et 3 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 3,7 g der produit que l'on chromatographie sur une colonne de 5 cm de diamètre contenant 400 g de silice (0,02—0,045 mm). On élue par de l'acétate d'éthyle sous une pression 0,4 bar (40 kPa) en recueillant des fractions der 400 $cm^3$. Les 8 premières fractions sont éliminées. Les 5 fractions suivantes sont réuines et concentrées à sec sous pression réduite (20 mm de mercure; 2,7 kPa) à une température voisine de 50°C. On obtient ainsi 1,9 g de produit brut. Ce produit est dissous dans 13 $cm^3$ d'acétonitrile bouillant. La solution obtenue est additionée de 0,5 g de noir décolorant et filtrée à chaud. Le filtrat obtenu est refroidi à une temperature voisine de 4°C pendant 16 heures. Les cristaux apparus sont séparés par filtration, lavés 2 fois par 6 $cm^3$ au total d'acétonitrile refroidi à une température voisine de 4°C et 2 fois par 20 $cm^3$ au total d'ether diéthylique puis séchés sous pression réduite (20 mm de mercure; 2,7 kPa) à une température voisine de 20°C en présence de potasse en pastilles. On obtient ainsi 1,4 g de N-[(pyridyl-2 carbonyl)-3 phényl] (pyridyl-3)-3 1H,3H-pyrrolo [1,2-c] thiazolecarboxamide-7 sous forme de cristaux crème fondant à 165°C.

Le chlorhydrate de chloroformyl-7 (pyridyl-3)-3 1H,3H-pyrrolo [1,2-c] thiazole est préparé selon la méthode décrite dans le brevet européen n° 0 115 979.

L'(amino-3 benzoyl)-2 pyridine peut être préparée de la façon suivante: A une suspension de 19 g de (nitro-3 benzoyl)-2 pyridine dans 360 $cm^3$ d'une solution d'éthanol chlorhydrique 3,7N, on ajoute en 45 minutes à une température voisine de 3°C, 63 g de chlorure stanneux, a l'état de dihydrate. La suspension obtenue est agitée à une température voisine de 4°C pendant 1 heure et 30 minutes, à une température voisine de 20°C pendant 1 heure et 30 minutes puis à une température voisine de 80°C pendant 1 heure et 30 minutes. Le solvant est évaporé sous pression réduite (20 mm de mercure; 2,7 kPa) à une température voisine de 50°C. Le résidu est repris par 200 $cm^3$ d'eau distillée. La solution aqueuse est amenée à un pH voisin de 11 par addition d'une solution aqueuse de soude 10N à une température voisine der 25°C, saturée par du chlorure de sodium puis est extraite 3 fois par 600 $cm^3$ au total d'ether diethylique. Les extraits éthérés sont réunis, lavés 3 fois par 300 $cm^3$ au total d'une solution aqueuse saturée de chlorure de sodium, séchés sur du sulfate de magnésium anhydre, additionnés de 0,5 g de noir décolorant, filtrés et concentrés à sec sous pression réduite (20 mm de mercure; 2,7 kPa) à une température voisine de 40°C. On obtient 15,8 g de produit brut que l'on chromatographie sur une colonne de 8 cm de diamètre contenant 500 g de silice (0,02—0,045 mm). On élue par un mélange de cyclohexane et d'acétate d'éthyle (50—50 en volumes) sous un pression de 0,4 bar (40 kPa) en recueillant des fractions de 400 $cm^3$. Les 7 premières fractions sont éliminées. Les 7 fractions suivantes sont réunies et concentrées à sec sous pression réduite (20 mm de

mercure; 2,7 kPa) à une température voisine de 50°C. On obtient 12 g d'(amino-3 benzoyl)-2 pyridine sous forme d'une huile rouge employée brute dans les synthèses ultérieures.

La (nitro-3 benzoyl)-2 pyridine peut être préparée d'après A. R. HANDS et A. R. KATRITZKY, J. Chem. Soc., 1754 (1958).

Exemple 20

A une solution de 4,1 g de (thénoyl-2)-3 aniline et de 4,05 g de triéthylamine dans 150 $cm^3$ de dioxanne chauffée à une température voisine de 70°C, on ajoute en 10 minutes à une température comprise entre 70 et 82°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 350 $cm^3$ de chlorure de méthylène. La solution obtenue est lavée 2 fois par 300 $cm^3$ au total d'eau distillée, 2 fois par 300 $cm^3$ au total d'une solution aqueuse de soude 2N, et 2 fois par 300 $cm^3$ au total d'eau distillée puis séchée sur du sulfate de magnésium anhydre, additionnée de 0,5 g de noir décolorant, filtrée et concentrée à sec sous pression réduite (20 mm mercure; 2,7 kPa) à une température voisine de 60°C. On obtient ainsi 10 g de produit brut que l'on dissout dans 250 $cm^3$ d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 50 $cm^3$ au total d'acétonitrile et 3 fois par 90 $cm^3$ au total d'éther diéthylique puis séchés sous pression réduite (20 mm de mercure; 2,7 kPa) à une température voisine de 20°C en présence de potasse en pastilles. On obtient ainsi 5 g de N-[(thénoyl-2)-3 phényl] (pyridyl-3)-3 1H,3H-pyrrolo [1,2-c] thiazolecarboxamide-7 sous forme de cristaux crème fondant à 172°C.

Le chlorhydrate de chloroformyl-7 (pyridyl-3)-3 1H,3H-pyrrolo [1,2-c] thiazole est préparé selon la méthode décrite dans le brevet européen n° 0 115 979.

La (thénoyl-2)-3 aniline peut être préparée de la façon suivante: A une suspension de 6,8 g de (thénoyl-2)-3 nitrobenzène dans 130 $cm^3$ d'une solution d'éthanol chlorhydrique 3,7N, on ajoute en 40 minutes, à une température voisine de 4°C, 22,8 g de chlorure stanneux a l'état de dihydrate. Après agitation à une température voisine de 4°C pendant 1 heure puis à une température voisine de 20°C pendant 1 heure, la solution obtenue est chauffée à une température voisine de 78°C pendant 1 heure. Le solvant est évaporé sous pression réduite (20 mm de mercure; 2,7 kPa) à une température voisine de 50°C. Le résidu obtenu est repris par un mélange de 50 $cm^3$ d'eau distillée et de 100 $cm^3$ d'éther diéthylique auquel on ajoute, à une température voisine de 15°C, 130 $cm^3$ d'une solution aqueuse de soude 10N. La phase organique est séparée et la phase aqueuse est extraite 4 fois par 400 $cm^3$ au total d'éther diéthylique. Les extraits éthérés sont réunis, lavés 3 fois par 300 $cm^3$ au total d'eau distillée, séchés sur du sulfate de magnésium anhydre, additionnés de 0,5 g de noir décolorant, et concentrés à sec sous pression réduite (20 mm de mercure; 2,7 kPa) à une température voisine de 40°C. On obtient ainsi 5,7 g de (thénoyl-3)-2 aniline sous forme de cristaux jaunes fondant à 105°C.

Le (thénoyl-3)-2 nitrobenzène peut être préparé d'après R. GONCALVES, M. R. KEGELMAN et E. V. BROWN, J. Org. Chem., *17,* 705 (1952).

Exemple 21

A une solution de 3,7 g d'anilino-3 aniline et de 4,65 g de triéthylamine dans 150 $cm^3$ de dioxanne chauffée à une température voisine de 65°C, on ajoute en 5 minutes à une température comprise entre 65 et 70°C, 6 g de chlorhydrate de chloroformyl-7 (pyridyl-3)-3 1H,3H-pyrrolo [1,2-c] thiazole. La suspension obtenue est chauffée sous agitation à une température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau solution distillée, 2 fois par 300 $cm^3$ au total d'une solution aqueuse de soude 2N et 2 fois par 300 $cm^3$ au total d'eau distillée puis séchée sur du sulfate de magnésium anyhydre, additionnée de 0,5 g de noir décolorant, filtrée et concentrée à sec sous pression réduite (20 mm de mercure; 2,7 kPa) à une température voisine de 60°C. On obtient ainsi 8,4 g de produit brut que l'on dissout dans 50 $cm^3$ d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 45 $cm^3$ au total d'acétonitrile et 3 fois par 90 $cm^3$ au total d'ether diéthylique puis séchés sous pression réduite (20 mm de mercure; 2,7 kPa) à une température voisine de 20°C en présence de potasse en pastilles. On obtient ainsi 0,3 g de produit fondant à 140°C. Les liqueurs-mères de cristallisation sont concentrées à sec sous pression réduite (20 mm de mercure; 2,7 kPa) à une température voisine de 60°C. On obtient ainsi 7,5 g de produit que l'on chromatographie sur une colonne de 6 cm de diamètre contenant 480 g de silice (0,02—0,045 mm) en éluant par un mélange d'acétate d'éthyle et de cyclohexane (80—20 en volumes) sous une pression de 0,4 bar (40 kPa) et en recueillant des fractions de 200 $cm^3$. Les 9 premiéres fractions sont éliminées. Les 9 fractions suivantes sont réunies et concentrées à sec sous pression réduite (20 mm de mercure; 2,7 kPa) à une température voisine de 50°C. On obtient ainsi 5,2 g de produit que l'on réunit au lot provenant de la première cristallisation et que l'on dissout dans 55 $cm^3$ d'acétonitrile bouillant. La solution obtenue est

additionnée de 0,5 g de noir décolorant et filtrée à chaud. Le filtrat est refroidi à une température voisine de 4°C pendant 16 heures. Les cristaux apparus sont séparés par filtration, lavés 3 fois par 30 $cm^3$ au total d'acétonitrile refroidi à une température voisine de 4°C et 3 fois par 90 $cm^3$ au total d'éther diéthylique puis séchés sous pression réduite (20 mm de mercure; 2,7 kPa) à une température voisine de 20°C en présence de potasse en pastilles. On obtient ainsi 2,4 g de N-(anilino-3 phényl) (pyridyl-3)-3 1H,3H-pyrrolo [1,2-c] thiazolecarboxamide-7 sous forme de cristaux blancs fondant à 158°C.

L'anilino-3 aniline peut être préparée selon H. WIELAND et W. RHEINHEIMER, Annalen, *423,* 1 (1931).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 22

A une solution de 3,5 g de benzyl-3 aniline et de 3,9 de triéthylamine dans 100 $cm^3$ de dioxanne chauffée à une température voisine de 60°C, on ajoute en 25 minutes à une température comprise entre 60 et 65°C, 5,7 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 60°C. Le résidu est dissous dans 250 $cm^3$ de chlorure de méthylène. La solution obtenue est lavée 2 fois par 160 $cm^3$ au total d'eau distillée, 2 fois par 200 $cm^3$ au total d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 5 g de produit brut que l'on dissout dans 50 $cm^3$ d'acétonitrile boillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés par 10 $cm^3$ d'acétonitrile refroidi à une température voisine de 4°C et 2 fois par 20 $cm^3$ au total d'éther diéthylique puis séchés sous pression réduite (20 mm de mercure; 2,7 kPa) à une température voisine de 20°C en présence de potasse en pastilles. On obtient ainsi 2,4 g de N-(benzyl-3 phényl) (pyridyl-3)-3 1H,3H-pyrrolo [1,2-c] thiazolecarboxamide-7 sous forme de cristaux blancs fondant à 139,5°C.

La benzyl-3 aniline peut être préparée selon H. OELSCHLAGER, Chem. Ber., *89,* 2025 (1956).

Le chlorhydrate de chloroformyl-7 (pyridyl-3)-3 1H,3H-pyrrolo [1,2-c] thiazole est preparé selon la méthode décrite dans le brevet européen n° 0 115 979.

Exemple 23

A une solution de 6 g de phénylthio-3 aniline et de 6,1 g de triéthylamine dans 200 $cm^3$ de dioxanne chauffée à une température voisine de 60°C, on ajoute en 5 minutes à une température comprise entre 60 et 68°C, 9 g de chlorhydrate de chloroformyl-7 (pyridyl)-3)-3 1H,3H-pyrrolo [1,2-c] thiazole. La suspension obtenue est chauffée sous agitation à une température voisine de 100°C pendant 7 heures puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée 2 fois par 300 $cm^3$ au total d'eau distillée, 2 fois par 300 $cm^3$ au total d'une solution aqueuse de soude 2N, et 2 fois par 300 $cm^3$ au total d'eau distillée puis séchée sur de sulfate de magnésium anhydre, additionnée de 0,5 g de noir décolorant, filtrée et concentrée à sec sous pression réduite (20 mm de mercure; 2,7 kPa) à une température voisine de 60°C. On obtient ainsi 11,5 g de produit brut que l'on dissout dans 110 $cm^3$ d'acétonitrile boillant. La solution obtenue est additionnée de 0,5 g de noir décolorant et filtrée à chaud. Le filtrat est refroidi à une température voisine de 10°C pendant 1 heure. Les cristaux apparus sont separés par filtration, lavés 3 fois par 45 $cm^3$ au total d'acétonitrile et 3 fois par 90 $cm^3$ au total d'éther diethylique puis séchés sous pression réduite (20 mm de mercure; 2,7 kPa) à une température voisine de 20°C en présence de potasse en pastilles. On obtient ainsi 7,2 g de N-(phénylthio-3 phényl) (pyridyl-3)-3 1H,3H-pyrrolo [1,2-c] thiazolecarboxamide-7 sous forme de cristaux crème fondant à 152°C.

La phénylthio-3 aniline peut être préparée selon la méthode décrite dans le brevet belge n° 765 558.

Le chlorhydrate de chloroformyl-7 (pyridyl)-3)-3 1H,3H-pyrrolo [1,2-c] thiazole est préparé selon la méthode décrite dans le brevet européen n° 0 115 979.

Exemple 24

A une solution de 4 g d'(amino-3 benzoyl)-4 pyridine et de 4,05 g de triéthylamine dans 100 $cm^3$ de dioxanne chauffée à une température voisine de 65°C, on ajoute en 15 minutes à une température comprise entre 65 et 70°C, 6 g de chlorhydratte de chloroformyl-7 (pyridyl-3)-3 1H,3H-pyrrolo [1,2-c] thiazole. La suspension obtenue est chauffée sous agitation à une température voisine de 100°C pendant 6 heures et 30 minutes puis est agitée à une température voisine de 20°C pendant 16 heures. Le solvant est évaporé sous pression réduite (20 mm de mercure; 2,7 kPa) à une température voisine de 60°C. Le résidu est dissous dans 300 $cm^3$ de chlorure de méthylène. La solution obtenue est lavée par 100 $cm^3$ d'eau distillée, par 100 $cm^3$ d'une solution aqueuse de soude N et 5 fois par 500 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 6,5 g de

produit brut que l'on dissout dans 300 $cm^3$ d'acétonitrile boillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 2 fois par 20 $cm^3$ au total d'acétonitrile refroidi à une température voisine de 4°C et 2 fois par 40 $cm^3$ au total d'ether diéthylique puis séchés sous pression réduite (20 mm de mercure; 2,7 kPa) à une température voisine de 20°C en présence de potasse en pastilles. On obtient ainsi 5,5 g de N-(isonicotinoyl-3 phényl) (pyridyl-3)-3 1H,3H-pyrrolo [1,2-c] thiazolecarboxamide-7 sous forme de cristaux blancs fondant à 186°C.

L'(amino-3-benzoyl)-4 pyridine peut être préparée d'après F. SAUTER, P. STANETTY et A. MESBAH, Monatsh., *107*, 1449 (1976).

Le chlorhydrate de chloroformyl-7 (pyridyl-3)-3 1H,3H-pyrrolo [1,2-c] thiazole est préparé selon la méthode décrite dans le brevet européen n° 0 115 979.

Exemple 25

A une solution de 4,1 g d'amino-3 chloro-4 benzophénone et de 3,65 g de triéthylamine dans 80 $cm^3$ de dioxanne chauffée à une température voisine de 60°C, on ajoute en 15 minutes à une température comprise entre 60 et 62°C, 5,4 g de chlorhydrate du chlorure d'acide provenant de l'acide (+) (pyridyl-3)-3 1H,3H-pyrrolo [1,2-c] thiazole carboxylique-7. La suspension obtenue est chauffée sous agitation à une température voisine de 100°C pendant 5 heures et 40 minutes puis est agitée à une température voisine de 20°C pendant 16 heures. Le solvant est évaporé sous pression réduite (20 mm de mercure; 2,7 kPa) à une température voisine de 50°C. Le résidu est dissous dans 250 $cm^3$ de chlorure de méthylène. La solution obtenue est lavée 2 fois par 200 $cm^3$ au total d'eau distillée, par 100 $cm^3$ d'une solution aqueuse de soude 2N, et 3 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 45°C. On obtient ainsi 8,3 g de produit brut que l'on dissout dans 50 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 30 $cm^3$ au total d'acétonitrile refroidi à une température voisine de 4°C et 2 fois par 60 $cm^3$ au total d'éther diéthylique puis séchés sous pression réduite (20 mm de mercure; 2,7 kPa) à une température voisine de 20°C en présence de potasse en pastilles. On obtient ainsi 3,7 g (+)N-(chloro-2-benzoyl-5 phényl) (pyridyl-3)-3 1H,3H-pyrrolo [1,2-c] thiazolecarboxamide-7 sous forme de cristaux crème fondant à 154°C.

$[\alpha]_J^{20} = +48,5° \pm 0,8°$ (c = 0,86; diméthylformamide).

Le chlorhydrate de (+) chloroformyl-7 (pyridyl-3)-3 1H,3H-pyrrolo [1,2-c] thiazole est préparé comme décrit à l'exemple 2.

L'amino-3 chloro-4 benzophénone peut être préparée selon D. MARON et C. FOX, Ber., *47*, 2774 (1914).

Exemple 26

A une solution de 3,4 g d'amino-3'méthoxy-2 benzophénone et de 3,05 g de triéthylamine dans 90 $cm^3$ de dioxanne chauffée à une température voisine de 62°C, on ajoute en 15 minutes à une température comprise entre 62 et 69°C, 4,5 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures puis est agitée à une température voisine de 20°C pendant 16 heures. Le solvant est évaporé sous pression réduite (20 mm de mercure; 2,7 kPa) à une température voisine de 50°C. Le résidu est dissous dans 250 $cm^3$ de chlorure de méthylène. La solution obtenue est lavée par 100 $cm^3$ d'eau distillée, 2 fois par 200 $cm^3$ au total d'une solution aqueuse de soude N et 3 fois par 300 $cm^3$ au total d'eau distillée puis séchée sur du sulfate de magnésium anhydre, additionnée de 0,5 g de noir décolorant, filtrée et concentrée à sec sous pression réduite (20 mm de mercure; 2,7 kPa) à une température voisine de 45°C. On obtient ainsi 6,8 g de produit brut que l'on dissout dans 60 $cm^3$ d'acétonitrile bouillant. La solution obtenue est additionnée de 0,5 g de noir décolorant et filtrée à chaud. Le filtrat est refroidi à une température voisine de 4°C pendant 1 heure. Les cristaux apparus sont séparés par filtration, lavés 2 fois par 30 $cm^3$ au total d'acétonitrile refroidi à une température voisine de 4°C et 3 fois par 60 $cm^3$ au total d'éther diéthylique puis séchés sous pression réduite (20 mm de mercure; 2,7 kPa) à une température voisine de 20°C en présence de potasse en pastilles. On obtient ainsi 4,1 g de N-[méthoxy-2 benzoyl)-3 phényl] (pyridyl-3)-3 1H,3H-pyrrolo [1,2-c] thiazolecarboxamide-7 sous forme de critaux blancs fondant à 184°C.

Le chlorhydrate de chloroformyl-7 (pyridyl-3)-3 1H,3H-pyrrolo [1,2-c] thiazole est préparé selon la méthode décrite dans le brevet européen n° 0 115 979.

L'amino-3' méthoxy-2 benzophénone peut être préparée de la façon suivante: A une suspension de 3,9 g de méthoxy-2 nitro-3' benzophénone dans un mélange de 40 $cm^3$ d'éthanol et de 15,2 $cm^3$ d'acide chlorhydrique concentré (11,7N) chauffée à une température voisine de 62°c, on ajoute en 5 minutes à une température comprise entre 62 et 66°C, 10,3 g de chlorure stanneux à l'état de dihydrate. La solution obtenue est chauffée à une température voisine de 80°C pendant 4 heures, additionnée de 1 g de chlorure stanneux à l'état de dihydrate et chauffée à une température voisine de 80°C pendant 1 heure supplémentaire puis agitée à une température voisine de 20°C pendant 16 heures. Le solvant est évaporé sous pression réduite (20 mm de mercure; 2,7 kPa) à une température voisine de 50°C. Le résidu est dissous dans 40 $cm^3$ d'eau distillée et la solution obtenue est amenée, en conservant la température au voisinage

de 10°C, à un pH voisin de 13 par addition de 50 $cm^3$ d'une solution aqueuse de soude 10N puis est extraite 3 fois par 150 $cm^3$ au total de chlorure de méthylène. Les extraits organiques sont réunis, lavés 2 fois par 200 $cm^3$ au total d'eau distillée, séchés sur du sulfate de magnésium anhydre, additionnés de 0,5 g de noir décolorant, filtrés et concentrés à sec sous pression réduite (200mm de mercure; 2,7 kPa) à une température voisine de 40°C. On obtient ainsi 3,4 g d'amino-3'méthoxy-2 benzophénone sous forme de cristaux jaune verdatre fondant à 81°C.

La méthoxy-2 nitro-3' benzophénone peut être préparée de la façon suivante: Une suspension de 3,6 g d'hydroxy-2 nitro-3' benzophénone, de 4 g de carbonate de potassium et de 4,2 g d'iodure de méthyle dans 100 $cm^3$ d'acétone est chauffée à une température voisine de 56°C pendant 16 heures. Le solvant est évaporé sous pression réduite (20 mm de mercure; 2,7 kPa) à une température voisine de 40°C. Le solide obtenu est dissous dans un mélange de 80 $cm^3$ d'eau distillée et de 50 $cm^3$ d'acétate d'éthyle. La phase organique est séparée et la phase aqueuse est extraite 2 fois par 100 $cm^3$ au total d'acétate d'éthyle. Les extraits organiques sont réunis, lavés 3 fois par 90 $cm^3$ au total d'eau distillée, séchés sur du sulfate de magnésium anhydre, additionnés de 0,5 g de noir décolorant, filtrés et concentrés à sec sous pression réduite (20 mm de mercure; 2,7 kPa) à une température voisine de 50°C. On obtient 3,8 g de produit brut que l'on met en suspension dans 25 $cm^3$ d'oxyde d'isopropyle à une température voisine de 4°C. Les cristaux apparus sont sépares par filtration, lavés 2 fois par 10 $cm^3$ au total d'oxyde d'isopropyle refroidi à une température voisine de 4°C puis séchés sous pression réduite (20 mm de mercure; 2,7 kPa) à une température voisine de 20°C en présence de potasse en pastilles. On obtient ainsi 3,3 g de méthoxy-2 nitro-3' benzophénone sous forme de cristaux beige fondant à 94°C.

L'hydroxy-2 nitro-3' benzophénone peut être obtenue selon I. H. BOWEN et J. R. LEWIS, J. Chem. Soc., Perkin Trans. I, 683 (1972).

Exemple 27

A une solution de 6,57 g de cinnamoyl-3 aniline et de 5,95 g de triéthylamine dans 165 $cm^3$ de dioxanne chauffée à une température voisine de 60°C, on ajoute en 10 minutes à une température comprise entre 60 et 75°C, 8,8 g de chlorhydrate de chloroformyl-7 (pyridyl-3)-3 1H,3H-pyrrolo [1,2-c] thiazole. La suspension obtenue est chauffée sous agitation à une température voisine de 100°C pendant 6 heures et 30 minutes puis est agitée à une température voisine de 20°C pendant 16 heures. Le solvant est évaporé sous pression réduite (20 mm de mercure; 2,7 kPa) à une température voisine de 50°C. Le résidu est dissous dans 500 $cm^3$ de chlorure de méthylène. La solution obtenue est lavée 4 fois par 650 $cm^3$ au total d'eau distillée, puis séchée sur du sulfate du sulfate de magnésium anhydre, additionnée de 0,5 g de noir décolorant, filtrée et concentrée à sec sous pression réduite (20 mm de mercure; 2,7 kPa) à une température voisine de 50°C. On obtient ainsi 13,5 g de produit brut que l'on dissout dans 300 $cm^3$ d'acétonitrile bouillant. La solution obtenue est additionnée de 0,5 g de noir décolorant et filtrée à chaud. Le filtrat est refroidi à une température voisine de 4°C pendant 16 heures. Les cristaux apparus sont séparés par filtration, lavés 3 fois par 60 $cm^3$ au total d'acétonitrile puis séchés sous pression réduiter (20 mm de mercure; 2,7 kPa) à une température voisine de 20°C en présence de potasse en pastilles. On obtient ainsi 7,1 g de N-(cinnamoyl-3 phényl) (pyridyl-3)-3 1H,3H-pyrrolo [1,2-c] thiazolecarboxamide-7 sous forme de cristaux beiges fondant à 190°C.

La cinnamoyl-3 aniline peut être préparée selon W. DAVEY et J. R. GWILT, J. Chem., Soc., 1008 (1957).

Le chlorhydrate de chloroformyl-7 (pyridyl-3)-3 1,3H-pyrrolo [1,2-c] thiazole est préparé selon la méthode décrite dans le brevet européen n° 0 115 979.

La présente invention concerne également les médicaments constitués par un produit de formule générale (I), sous forme libre ou sous forme de sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être utilisés par voie orale, parentérale, rectale ou topique.

Comme compositions solides pour administration orale peuvent être utilisés des comprimés, pilules, poudres (notamment dans des capsules de gélatine ou des chachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple ou un plusieurs lubrifiants tel que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables.

Ces compositions peuvent également contenir des adjuvants, en particulier des agents mouillants,

isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement de toutes les conditions pathologiques dans lesquelles le PAF-acéther peut être incriminé directement ou indirectement, notamment les affections allergiques, inflammatoires et les affections du système digestif tels que les ulcères, les colites et les lésions intestinales causées par les irradiations ou les chocs endotoxiniques.

Les doses dépendent de l'éffet recherché et de la durée du traitement; elles sont généralement comprises entre 25 et 300 mg par jour par voie orale, intraveineuse ou par inhalation pour un adulte en une ou plusieurs prises.

D'une façon générale, le médicin déterminera la posologie qu'il estime la plus approprié en fonction de l'âge, du poids et de tous les autres facteurs propres au sujet à traiter.

Les exemples suivants, donnés à titre non limitatif, illustrent des compositions selon l'invention.

Exemple A

On prépare, selon la technique habituelle, des comprimés dosés à 25 mg de produit actif ayant la composition suivante:

| | |
|---|---|
| N-(phénoxy-3 phényl) (pyridyl-3)-3 1H,3H-pyrrolo [1,2-c] thiazolecarboxamide-7 | 25 mg |
| amidon | 60 mg |
| lactose | 50 mg |
| stéarate de magnésium | 2 mg |

Exemple B

On prépare, selon la technique habituelle, des comprimés dosés à 25 mg de produit actif ayant la composition suivante:

| | |
|---|---|
| (+)N-(benzoyl-3 phényl) (pyridyl-3)-3 1H,3H-pyrrolo [1,2-c] thiazolecarboxamide-7 | 25 mg |
| amidon | 60 mg |
| lactose | 50 mg |
| stéarate de magnésium | 2 mg |

Exemple C

On prépare, selon la technique habituelle, une solution injectable contenant 5 mg de produit actif ayant la composition suivante:

| | |
|---|---|
| N-[(pyridyl-3 oxy) phényl] (pyridyl-3)-3 1H,3H-pyrrolo [1,2-c] thiazolecarboxamide-7 | 5 mg |
| solution d'acide chlorhydrique 0,1N | 0,25 $cm^3$ |
| soluté injectable q.s.p. | 2 $cm^3$ |

**Revendications pour les Etats contractants: BE CH DE FR GB IT LI LU NL SE**

1. Nouveau dérivé du 1H,3H-pyrrolo [1,2-c] thiazole caractérisé en ce qu'il répond à la formule générale:

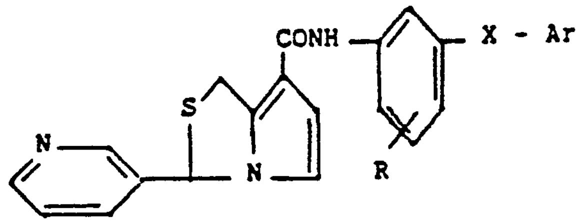

(I)

dans laquelle R représente un atome d'hydrogène ou d'halogène ou un radical alcoyloxy, X représente un atome d'oxygène ou de soufre ou un radical imino, carbonyle, carbonylméthyléne, ou vinylènecarbonyle ou bien représente un radical méthylène et Ar représente un radical phényle, naphtyle, pyridyle, thiényle, ces radicaux pouvant être non substitués ou bien porter un substituant choisi parmi les atomes d'halogène ou les radicaux alcoyle ou alcoyloxy, étant entendu que les radicaux alcoyle et portions alcoyle contiennent 1 à 4 atomes de carbone en chaîne droite ou ramifiée et que l'invention concerne les produits racémiques, les énantiomères dus à la présence d'un carbone asymétrique en position 3 du cycle pyrrolethiazole et les mélanges de ces énantiomères, ainsi que sels pharmaceutiquement acceptables.

2. Procédé de préparation de nouveaux dérivés selon la revendication 1, caractérisé en ce que l'on fait agir une amine de formule générale:

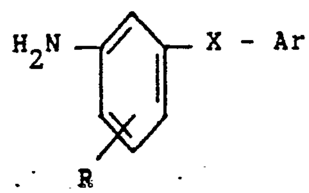

(II)

dans laquelle R, X et Ar sont définis comme à la revendication 1, sur un acide racémique ou optiquement actif de formule:

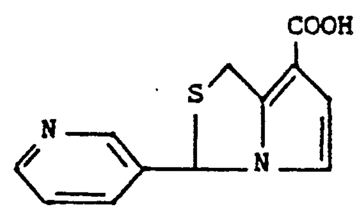

(III)

ou un dérivé réactif de cet acide, puis isole le produit obtenu et le transforme éventuellement en un sel pharmaceutiquement compatible.

3. Procédé de préparation de nouveaux dérivés selon la revendication 1, caractérisé en ce que l'on fait réagir le produit de réaction du chlorure de p-toluènesulfonyle, de la triéthylamine et de l'acide de formule:

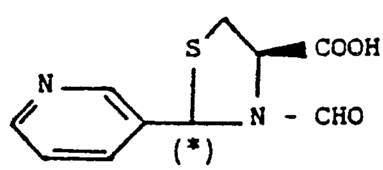

(V)

dans laquelle le symbole* représente le signe +, le signe − ou le signe ± selon qu'on met en oeuvre un produit dextrogyre, lévogyre ou racémique sur le produit de réaction de la triéthylamine et d'un produit de formule générale:

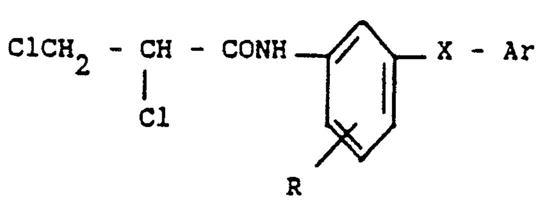

(VIII)

dans laquelle les symboles sont définis comme à la revendication 1, puis isole le produit obtenu et le transforme éventuellement en un sel pharmaceutiquement acceptable.

4. Composition pharmaceutique, caractérisé en ce qu'elle contient comme produit actif au moins un dérivé selon la revendication 1, en association avec un ou plusieurs diluants ou adjuvants compatibles et pharmaceutiquement acceptables.

**Revendication pour les Etats contractants: AT ES GR**

Procédé de préparation d'un nouveau dérivé du 1H,3H-pyrrolo [1,2-c] thiazole de formule générale:

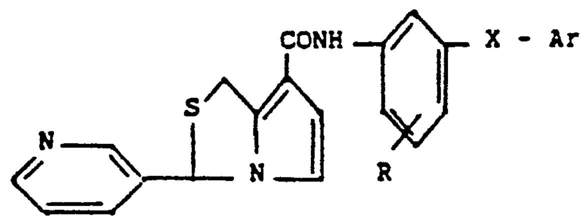

(I)

dans laquelle R représente un atome d'hydrogène ou d'halogène ou un radical alcoyloxy, X représente un atome d'oxygène ou de soufre ou un radical imino, carbonyle, carbonylméthyléne, ou vinylènecarbonyle ou bien représente un radical méthylène et Ar représente un radical phényle, naphtyle, pyridyle, thiényle, ces radicaux pouvant être non substitués ou bien porter un substituant choisi parmi les atomes d'halogène ou les radicaux alcoyle ou alcoyloxy, étant entendu que les radicaux alcoyle et portions alcoyle contient 1 à 4 atomes de carbone en chaîne droite ou ramifiée et que l'invention concerne les produits racémiques, les énantiomères dus à la présence d'un carbone asymétrique en position 3 du cycle pyrrolethiazole et les mélanges de ces énantiomères, ainsi que ses sels d'addition avec les acides pharmaceutiquement acceptables, caractérisé en ce que:

A — on fait réagir une amine de formule générale:

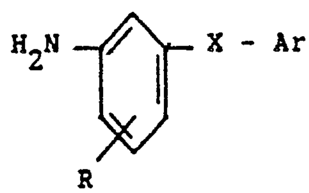

(II)

dans laquelle R, X et Ar sont définis comme précédemment sur un acide racémique ou optiquement actif de formule:

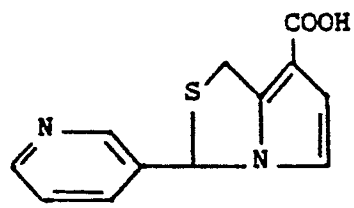

(III)

ou un dérivé réactif de cet acide, puis isole le produit obtenu et le transforme éventuellement en un sel pharmaceutiquement compatible, ou en ce que:

B — on fait réagir le produit de réaction du chlorure de p-toluène-sulfonique, de la triéthylamine et de l'acide de formule:

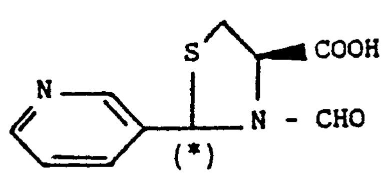

(V)

dans laquelle le symbole* représente le signe +, le signe − ou le signe ± selon qu'on met en oeuvre un produit dextrogyre, lévogyre ou racémique sur le produit de réaction de la triéthylamine et d'un produit de formule générale:

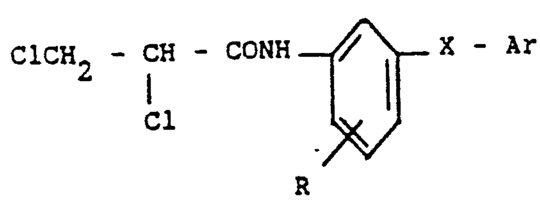

(VIII)

dans laquelle les symboles sont définis comme précédemment, puis isole le produit obtenue et le transforme éventuellement en un sel pharmaceutiquement acceptable.

**Patentansprüche für die Vertragsstaaten: BE CH DE FR GB IT LI LU NL**

1. Neues Derivat des 1H,3H-Pyrrolo[1,2-c]thiazols, dadurch gekennzeichnet, daß es der allgemeinen Formel:

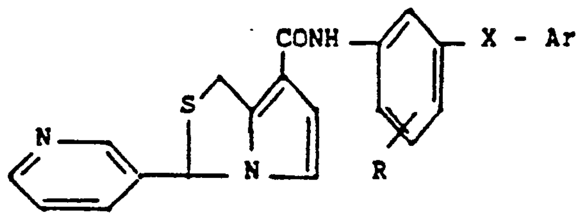

(I)

entspricht, worin R ein Wasserstoff- oder Halogenatom oder einen Alkyloxyrest bedeutet, X ein Sauerstoff- oder Schwefelatom oder einen Imino-, Carbonyl-, Carbonylmethylen- oder Vinylencarbonylrest oder auch einen Methylenerest bedeutet, und Ar einen Phenyl-, Naphthyl-, Pyridyl-, Thienylrest bedeutet, wobei diese Reste nicht-substituiert sein können oder einen Substituenten tragen, ausgewählt unter den Halogenatomen oder den Alkyl- oder Alkoxyresten, wobei die Alkylreste und Alkylteile 1 bis 4 Kohlenstoffatome in gerader oder verzweigter Kette enthalten und daß die Erfindung die racemischen Produkte, die aufgrund der Anwesenheit eines asymmetrischen Kohlenstoffatoms in 3-Stellung des Pyrrolothiazolrings vorhandenen Enantiomeren und die Mischungen dieser Enantiomeren betrifft sowie ihre pharmazeutisch annehmbaren Salze.

2. Verfahren zur Herstellung der neuen Derivate gemäß Anspruch 1, dadurch gekennzeichnet, daß man ein Amin der allgemeinen Formel:

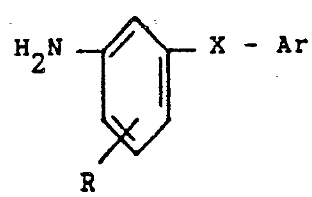

(II)

worin R, X und Ar wie in Anspruch 1 definiert sind, auf eine racemische oder optisch aktive Säure der Formel:

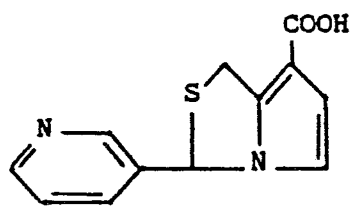

(III)

oder ein reaktives Derivat dieser Säure einwirken läßt, das erhaltene Produkt dann isoliert und es gegebenenfalls in ein pharmazeutisch verträgliches Salz überführt.

3. Verfahren zur Herstellung der neuen Derivate gemäß Anspruch 1, dadurch gekennzeichnet, daß man das Reaktionsprodukt des p-Toluolsulfonylchlorids, des Triethylamins und der Säure der Formel:

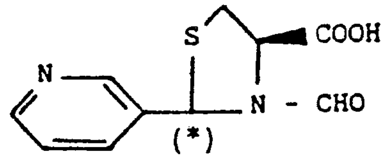

(V)

worin das Symbol* das Zeichen + oder − oder das Zeichen ± bedeutet, je nachdem, ob man ein rechtsdrehendes, linksdrehendes oder racemisches Produkt einsetzt, mit dem Reaktionsprodukt des Triethylamins und eines Produkts der allgemeinen Formel:

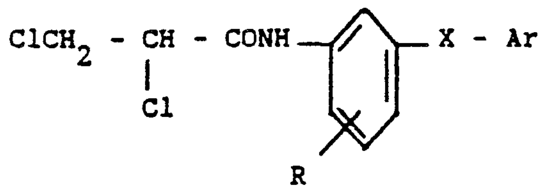

(VIII)

worin die Symbole wie in Anspruch 1 definiert sind, zur Reaktion bringt, das erhaltene Produkt dann isoliert und es gegebenenfalls in ein pharmazeutisch annehmbares Salz überführt.

4. Pharmazeutische Zusammensetzung, dadurch gekennzeichnet, daß sie als aktives Produkt mindestens ein Derivat gemäß Anspruch 1 enthält, zusammen mit einem oder mehreren verträglichen und pharmazeutisch annehmbren Verdünnungs- oder Hilfsmitteln.

**Patentanspruch für die Vertragsstaaten: AT ES GR**

Verfahren zur Herstellung eines neuen 1H,3H-Pyrrolo[1,2-c]thiazolderivats der allgemeinen Formel:

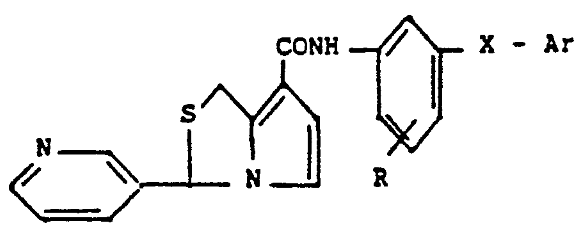

(I)

in welcher R einer Wasserstoff- oder Halogenatom oder einen Alkyloxyrest darstellt, X ein Sauerstoff- oder Schwefelatom oder einen Imino-, Carbonyl-, Carbonylmethylen- oder Vinylencarbonylrest darstellt oder auch einen Methylenrest darstellt und Ar einen Phenyl-,.Naphthyl-, Pyridyl-, Thienylrest darstellt, welche Reste unsubstituiert sein oder einen Substituenten tragen können, ausgewählt aus den Halogenatomen oder den Alkyl- oder Alkyloxyresten, wobei selbstverständlich die Alkylrete und Alkylteile 1 bis 4 Kohlenstoffatome in gerade oder verzweigter Kette enthalten und die Erfindung die racemischen Verbindungen, die auf die Anwesenheit eines asymmetrischen Kohlenstoffatoms in 3-Stellung des Pyrrolothiazolrings zurückzuführenden Enantiomeren und die Mischungen dieser Isomeren, sowie deren Salze mit pharmazeutisch zulässigen Säuren umfaßt, dadurch gekennzeichnet, daß man

A — ein Amin der allgemeinen Formel:

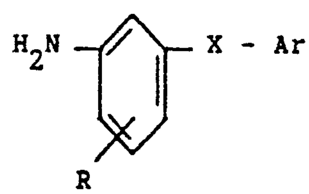

(II)

worin R, X und Ar die obige Bedeutung haben, mit einer racemischen oder optisch aktiven Säure der Formel:

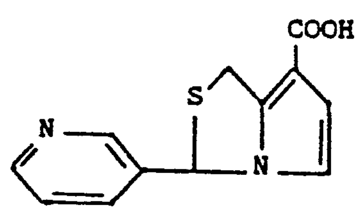

(III)

oder einem reaktionsfähigen Derivat dieser Säure umsetzt, dann die erhaltene Verbindung isoliert und sie gegebenenfalls in ein pharmazeutisch zulässiges Salz umwandelt, oder daß man

B — das Reaktionsprodukt von p-Toluolsulfonsäurechlorid, Triäthylamin und der Säure der Formel:

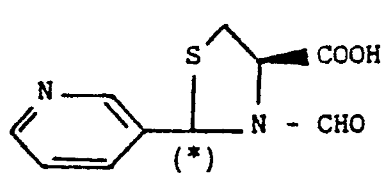

(V)

in welcher das symbol * das Zeichen +, das Zeichen – oder das Zeichen $\pm$ darstellt, je nachdem, ob man eine rechtsdrehende, linksdrehende oder racemische Verbindung einsetzt, mit dem Reaktionsprodukt von Triäthylamin und einer Verbindung der allgemeinen Formel:

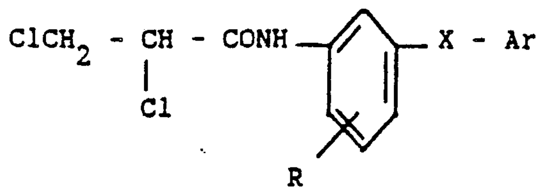

(VIII)

in welcher die Symbole die obige Bedeutung haben, reagieren läßt, dann die erhaltene Verbindung isoliert und sie gegebenenfalls in eine pharmazeutisch zulässiges Salz umwandelt.

**Claims for contracting States: BE CH DE FR GB IT LI LU NL SE**

1. A new 1H,3H-pyrrolo[1,2-c]thiazole derivative which is of the general formula:

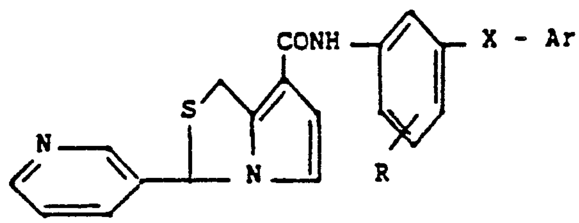

(I)

in which R represents a hydrogen or halogen atom or an alkyloxy radical, X represents an oxygen or sulphur atom or an imino, carbonyl, carbonylmethylene, or vinylene-carbonyl radical or alternatively represents a methylene radical and Ar represents a phenyl, naphthyl, pyridyl, or thienyl radical, it being possible for these radicals to be unsubstituted or to bear a substituent chosen from amongst halogen atoms or alkyl or alkyloxy radicals, it being understood that the alkyl radicals and the alkyl portions contain 1 to 4 carbon atoms in a straight- or branched-chain and that the invention relates to the racemic products, the enantiomers resulting from the presence of an asymmetric carbon atom in the 3-position of the pyrrolothiazole ring and the mixtures of these enantiomers, and to their pharmaceutically acceptable salts.

2. A method for the preparation of new derivatives according to claim 1, wherein an amine of general formula:

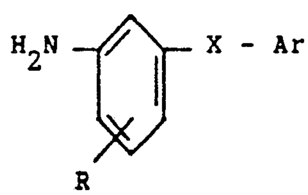

(II)

in which R, X and Ar are as defined in claim 1, is reacted with a racemic or optically active acid of formula:

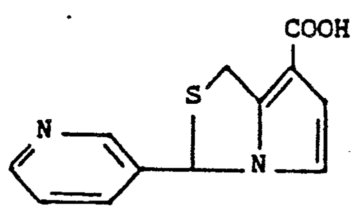

(III)

or a reactive derivative of this acid, the product obtained is then isolated and it is converted, if required, into a pharmaceutically compatible salt.

3. The method for the preparation of new derivatives according to claim 1, wherein the reaction product of p-toluenesulphonyl chloride, triethylamine and the acid of formula:

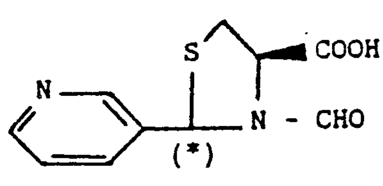

(V)

in which the symbol * represents the sign +, the sign − or the sign ± depending on whether the product employed is dextrorotatory, laevorotatory or racemic, is reacted with the reaction product of triethylamine and a product of general formula:

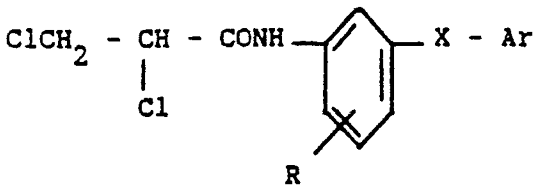

(VIII)

in which the symbols are as defined in the claim 1, the product obtained is then isolated and it is converted, if required, into a pharmaceutically acceptable salt.

4. A pharmaceutical composition which contains, as an active product, at least one derivative according to claim 1, combined with one or more compatible and pharmaceutically acceptable diluents or adjuvants.

**Claim for contracting States: AT ES GR**

A method for the preparation of a new 1H,3H-pyrrolo[1,2-c]thiazole derivative of general formula:

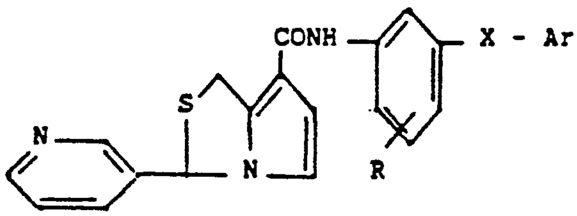

(I)

in which R represents a hydrogen or halogen atom or an alkyloxy radical, X represents an oxygen or sulphur atom or an imino, carbonyl, carbonylmethylene, or vinylene-carbonyl radical or alternatively represents a methylene radical and Ar represents a phenyl, naphthyl, pyridyl, or thienyl radical, it being possible for these radicals to be unsubstituted or to bear a substituent chosen from amongst halogen atoms or alkyl or alkyloxy radicals, it being understood that the alkyl radicals and the alkyl portions contain 1 to 4 carbon atoms in a straight- or branched-chain and that the invention relates to the racemic products, the enantiomers resulting from the presence of an asymmetric carbon atom in the 3-position of the pyrrolothiazole ring and the mixtures of these enantiomers, and to their pharmaceutically acceptable salts, wherein:

A — an amine of general formula:

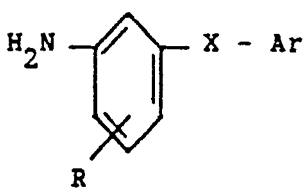

(II)

in which R, X and Ar are as defined above, is reacted with a racemic or optically active acid of formula:

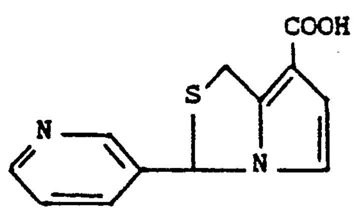

(III)

or a reactive derivative of this acid, the product obtained is then isolated and it is converted, if required, into a pharmaceutically compatible salt, or wherein:

B — the reaction product of p-toluenesulphonyl chloride, triethylamine and the acid of formula:

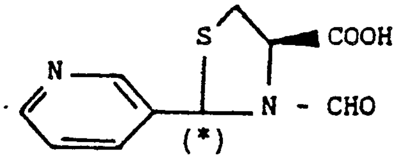

(V)

in which the symbol * represents the sign +, the sign − or the sign ± depending on whether the product employed is dextrorotatory, laevorotatory or racemic, is reacted with the reaction product of triethylamine and a product of general formula:

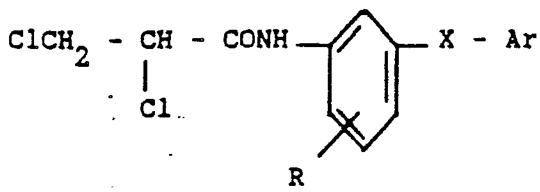

(VIII)

in which the symbols are as defined above, the product obtained is then isolated and it is converted, if required, into a pharmaceutically acceptable salt.